(19) Europäisches Patentamt
European Patent Office
Office européen des brevets

(11) Publication number: **0 447 442 B1**

(12) # EUROPEAN PATENT SPECIFICATION

(45) Date of publication of patent specification: **21.09.94**

(51) Int. Cl.$^5$: **C12Q 1/48**, C12Q 1/68, C12N 9/12

(21) Application number: **90900391.5**

(22) Date of filing: **29.11.89**

(86) International application number:
**PCT/SE89/00696**

(87) International publication number:
**WO 90/06373 (14.06.90 90/14)**

(54) **METHOD FOR POLYMERASE ACTIVITY DETERMINATION.**

(30) Priority: **30.11.88 SE 8804344**

(43) Date of publication of application:
**25.09.91 Bulletin 91/39**

(45) Publication of the grant of the patent:
**21.09.94 Bulletin 94/38**

(84) Designated Contracting States:
**BE CH DE ES FR GB IT LI NL SE**

(56) References cited:
**EP-A- 204 510**
**EP-A- 215 987**
**DE-A- 3 546 374**

**JOURNAL OF IMMUNOLOGICAL METHODS, vol. 84, 1985; M.D. TALBOT et al., pp. 165-175&NUM;**

**INT. JOURNAL OF BIOCHEMISTRY, vol. 17, no. 3, 1985; C.M. KLINGE et al., pp. 347-353&NUM;**

(73) Proprietor: **Källander, Clas Fredrik Runesson**
**Tunabergs Allé 4**
**S-753 37 Uppsala (SE)**

Proprietor: **Gronowitz, Jan-Simon**
**Sernanders väg 14**
**S-752 62 Uppsala (SE)**

(72) Inventor: **KÄLLANDER, Clas, Fredrik, Runesson**
**Tunabergs allé**
**S-753 37 Uppsala (SE)**
Inventor: **GRONOWITZ, Jan-Simon**
**Sernanders väg 14**
**S-752 62 Uppsala (SE)**
Inventor: **NEUMÜLLER, Thord, Magnus**
**Handarbetsvägen 24**
**S-752 57 Uppsala (SE)**
Inventor: **KARLSTRÖM, Rolf, Anders**
**Tegnérgatan 28A**
**S-753 27 Uppsala (SE)**
Inventor: **LANGSTRÖM-PERSSON, Ulla, M.**
**Hjälmsta, kerby**
**S-740 22 Bälinge (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**JOURNAL OF BIOLOGICAL CHEMISTRY, vol. 249, no. 4, 1974; K.Y. KU et al., pp. 1239-1246&NUM;**

Inventor: **WELTMAN, Eva, Helena**
**Petterslundsgatan 23C**
**S-753 28 Uppsala (SE)**
Inventor: **HAGSTRÖM, Asa, Margareta**
**Lästmakargatan 3B**
**S-754 34 Uppsala (SE)**

(74) Representative: **Kummelsten, Per-Arne**
**UPPSALA PATENTBYRA**
**P.O. Box 9013**
**S-750 09 Uppsala (SE)**

## Description

The present invention relates to a novel method of quantitatively determining polymerase activity.

Intracellular parasites, such as viruses, use the synthetic machinery of the cell for producing, based upon their own genetic information, specific parasitic elements instead of cell material. Among the elements encoded by the genome of the parasite are usually certain enzymes necessary for their replication, such as RNA or DNA polymerase or reverse transcriptase (also called RNA-dependent DNA polymerase), or essential for ensuring precursors for the production of genetic material, such as kinases, ribonucleotide reductase and deoxyribonuclease.

The immunological specificity of virally encoded enzymes as well as their relation to virus replication activity has made it interesting to design procedures for measuring such activities in clinical samples. This is especially true for the above mentioned polymerases. Thus, DNA polymerase activity may be detected in serum in connection with certain malign and viral diseases. The activity of reverse transcriptase has proved to be specifically characteristic for certain viruses involved in various neoplastic conditions, e.g., certain human leukemias, as well as for certain viral diseases, including the immunodeficiency disease AIDS and its preliminary stages caused by the human immunodeficiency virus HIV.

The determination of polymerase activity may thus be of value as a diagnostic method for detecting the presence of such diseases, monitoring the effect of the treatment of affected patients or monitoring patients after the treatment in respect of recurrences, such that the treatment may be started early before any clinical symptoms appear.

Generally, activity of DNA and RNA polymerases and reverse transcriptase may be determined by adding a suitably processed sample to a reaction solution in which the polymerase in question may exert its action and to this end contains a template of DNA- or RNA-type dissolved in the reaction solution, and a substrate in the form of a radio-labeled nucleoside triphosphate which is incorporated into the template by the activity of the polymerase, whereupon the radioactivity incorporated is measured. For DNA polymerase a double-stranded DNA material comprising single strand regions (i.e. provided with so-called nicks) may be used as template, while reverse transcriptase will act upon a single nucleotide chain of RNA-type, which on a part thereof must be hybridized with a so-called primer in the form a deoxyribonucleotide oligomer or a t-RNA, which is required to initiate the polymerization reaction catalyzed by the polymerase. As radioactive isotopes for labeling the substrates $^{3}H$ or $^{32}P$ have usually been used.

More specifically, US-A-3,755,086 discloses a method of detecting activity of reverse transcriptase in purified cell extract or plasma samples from mammals or birds, the template used being a polymer of RNA-type hybridized with a synthetic thymidine polynucleotide oligomer of 2-24 nucleotide units as a primer. In this method the purified sample is added to a reaction solution containing the template and $^{3}H$-labeled nucleoside triphosphate. After incubation the reaction is stopped and the template is precipitated by adding trichloroacetic acid (TCA) and taken up on a filter. The dried filter is then placed in a scintillation fluid, and the radioactivity incorporated into the template, which activity is a measure of the polymerase activity, is measured by liquid scintillation technique.

Similar methods specifically directed to HIV-determination via reverse transcriptase have been described inter alia by A. D. Hoffman et al., Virology 147, 326-335 (1985) and M. H. Lee et al., J. Clin. Microbiol.; Sept. 1987, pp. 1717-1721.

These conventional methods for the determination of reverse transcriptase activity, however, suffer from several disadvantages. Thus, the analysis can not be performed directly on clinical specimens due to the presence of disturbing factors, such as RNAses, phosphatases and competitive nucleotides. Also, the TCA-precipitation on a filter considerably restricts the sample volume that can be used, and thereby the sensitivity, the background in the scintillation determination of the radioactivity depending on the filter size. A further disadvantage is the use of $^{3}H$ as radioactive isotope, since, on one hand, it gives a low specific activity due to the relatively long half-life and can therefore not be used for low substrate concentrations, and, on the other hand, due to its type of radiation it gives problems with absorption of the radiation in the sample, so-called quenching. It additionally requires the use of a scintillation fluid for the activity determination, the possible clinical utility thereby being restricted. The above mentioned isotope $^{32}P$ which has also been used in similar contexts certainly has a high specific activity permitting it to be used at low substrate concentrations, but its short half-life (14 days) makes it unsuitable for practical use. The isotope $^{35}S$ which has also been used for these purposes will, due to its weak $\beta$-radiation, give considerable quenching problems.

Synthetic polynucleotides immobilized on a carrier, e.g. agarose, have previously been used for affinity purification of polymerases. Moreover, carrier-bound templates have been used for producing specific single-stranded DNA probes (P. L. Ashley et al., Anal. Biochem. 140, 95-103 (1984)). Further B. I. Milavetz

et al., Mol. Pharmacol., 13, 496-503 (1977), disclose the use of poly(A)-agarose as an immobilized template for identifying binding reactions taking place in the polymerization of DNA and investigating the inhibiting effect of various drugs (ansamycines) on DNA synthesis. For studying the drug inhibition, for example, the drugs dissolved in a buffer were passed through a column containing the polymerase bound to poly(A)-agarose hybridized with oligo$(dT)_{12-18}$ as primer and which had been produced by adsorbing the polymerase to the poly(A)-agarose and subsequently adding the primer. The polymerase activity of the column material was then determined by incubation in a reaction solution with $^3$H-deoxythymidine triphosphate ($^3$H-dTTP). After hydrolysis with NaOH and precipitation of the template/primer material with TCA, the radioactivity incorporated into the template was determined. Although the actual polymerization reaction was performed on the immobilized template, the subsequent radioactivity measurement thus required release of the template from the carrier and precipitation with TCA as in the above described determination method for polymerase activity.

Similar methods as described above for determining reverse transcriptase have been used for measuring DNA polymerase, i.e. by following the incorporation of a radioactive nucleotide into a free DNA template in solution by measurement after precipitation on a filter paper by TCA or ethanol. As template has usually been used nuclease treated, socalled activated, DNA and as radioactive isotope, $^3$H or $^{32}$P. The above discussed disadvantages of the hitherto used determination methods for reverse transcriptase therefore also concern the determination of DNA polymerase.

One purpose of the present invention is to provide an improved method of quantitatively determining polymerase activity, which method will eliminate most or all of the disadvantages of the prior art methods. Another purpose of the invention is to provide a kit for determining polymerase activity. These objects are achieved by a method and a kit, respectively, which have the features stated in the claims and which will be described in more detail hereinafter.

Thus, the invention in a first aspect thereof relates to a method of quantitatively determining polymerase activity in a sample by incubating the sample with a natural or completely or partially synthetic polynucleotide template and necessary substrates, including at least one radiolabeled nucleoside triphosphate complementary to the template, separating the template from the substrate and measuring the radioactivity incorporated into the template, which radioactivity is substantially proportional to the polymerase activity of the sample, said method being characterized in that the template is immobilized on a carrier and that the radiolabeled nucleoside triphosphate is labeled with a gamma-radiating isotope, and that the measurement of the incorporated radioactivity is performed directly on the immobilized template without release thereof from the carrier.

By using, in accordance with the invention, an immobilized template and measuring the radioactivity taken up directly on the immobilized template without any preceding release thereof several advantages are achieved. Thus, the previous sample volume limiting, and thereby sensitivity reducing, TCA precipitation on a filter is eliminated such that larger sample volumes may be used with a consequential increase of the sensitivity. By virtue of performing the measurement directly on the immobilized template also the technical analytical procedure will be simplified considerably. Further, the use of a gamma-radiating isotope will eliminate the previous problem of quenching and the need of a scintillation fluid.

According to a preferred embodiment of the invention the template is first incubated with the sample or specimen for incorporation of the polymerase present in the specimen into the template; the template with the affinity bound polymerase is then after washing contacted with the reagent solution containing the radiolabeled substrate and, when necessary, a primer, such as when the polymerase to be determined is reverse transcriptase. The primer may in that case be present on the template from the start or be added during the determination procedure.

By thus incubating the template with the specimen prior to the actual polymerase determination it has surprisingly been found that a polymerase determination may be performed directly on clinical specimens, such as sera and lymphocyte extracts taken directly from the patient. The disturbing factors, such as RNAses (in high concentration in cell extracts and also in cell supernatants) and competitive nucleotides, which occur in clinical specimens, thus appear to have a low affinity to the template.

As will be discussed in more detail further on the template may optionally be protected during the affinity binding of the polymerase, i.e. the "fish step", and/or the subsequent enzyme activity determination with suitable agents.

Both "semi-solid" materials, such as gels, and solid bodies may be used as carriers for immobilizing the template. Methods for coupling polynucleotides to various carriers are known and will not be described in any detail herein. As examples of gels may be mentioned agarose gels and Sepharose® (a bead-shaped agarose gel marketed by Pharmacia AB, Sweden). Commercially available immobilized templates that may be used for the purposes of the invention are, e.g., poly(A)-agarose (poly(A) covalently bound to CNBr-activated agarose; P-L Biochemicals, USA), poly(A)-Sepharose® (Pharmacia AB, Sweden). Exemplary of solid carriers are, e.g., balls, spheres or beads (also micro- or macrospheres) of plastic, e.g., polystyrene or polycarbonate spheres. A solid carrier suitable due to the simplified separation offered thereby is magnetic spheres or beads, e.g., tosylated magnetic Dynabeads (Dynal AS, Oslo, Norway).

The carrier-immobilized template to be used in each particular case will, of course, primarily depend on the polymerase to be determined. For determining reverse transcriptase the template should thus be a polynucleotide chain of RNA-type, in which case it may be a homopolymer, such as poly(rA), poly(rU), poly-(rG), poly(rC) and poly(rI), or a synthetic, semi-synthetic or natural copolymer built up from combinations thereof. While, in the case of a homopolymer only the corresponding complementary nucleoside triphosphate or analogue is required as substrate, a copolymer will, of course, require the use of complementary substrates to all the nucleotides; in the latter case only one of the substrates need, however, be labeled.

As previously mentioned a primer will be required to initiate the polymerization reaction in an assay for reverse transcriptase. In the present context primer means a nucleotide chain which may hybridize with the template, such as an oligonucleotide. For example, oligo(dT) will function as a primer for for poly(rA), and oligo(dG) for poly(rC). Depending upon the origin thereof different reverse transcriptases may exhibit different preferences for different template/primer combinations.

Since DNA polymerases act naturally upon templates in the form of double stranded DNA chains provided with so-called nicks, i.e. holes in one of the strands, the template supported by the carrier may in this case consist of a natural, semi-synthetic or synthetic double nucleotide chain of DNA-type provided with single strand regions. Also in this case the nucleotide chains may be homo- or copolymers. Alternatively, a single nucleotide chain having at least one double stranded region composed of long strand DNA and a short strand of either RNA or DNA may function as a template.

As immobilized template materials for RNA polymerases a double stranded natural or synthetic or semi-synthetic DNA chain may be used. In this case no single strand regions are required, but a primer may sometimes be necessary depending on the particular RNA polymerase. For RNA-dependent RNA polymerases immobilized RNA, "primed" in suitable manner will, of course, be required.

Since RNAses (e.g. in lymphocytes) will cleave phosphoester bonds, the template has suitably been chemically modified to completely or partially prevent cleavage. This may, e.g., be achieved by 2'-0-methylation (poly(Cm) etc.), P-methylation or P-sulphonation. Such modification of polynucleotides is per se previously known, see e.g. Murray and Atkinson, Biochem. 7(11), 4023-4029 (1968), the disclosure of which is incorporated herein by reference.

The substrate to be used for the polymerase catalyzed polymerization reaction will depend on the template used and should thus comprise nucleoside triphosphates or analogues complementary to all the nucleotides present in the template. As mentioned above, in accordance with the invention, at least one nucleoside triphosphate is labeled with a gamma-radiating radioactive isotope.

The gamma-radiating isotope is preferably one having a half-life in the range of about 20 days to 1 year. Particularly suitable for the purposes of the invention are iodine isotopes, preferably $^{125}I$, having a half-life of 60 days and a specific activity exceeding 2000 Ci/mMole.

Due to the use of a gamma-radiating isotope quenching problems and the need of a scintillation fluid are eliminated, and measurement directly on the immobilized template in accordance with the inventive method is rendered possible.

A particularly suitable radiolabeled substrate for the purposes of the invention is $^{125}I$-5-iodine-2'-deoxyuridine triphosphate, hereinafter referred to as $^{125}I$-IUdRTP. In addition to $^{125}I$ having an appropriate half-life for the purposes, being practical to handle and simple to measure due to its gamma-radiation, IUdRTP has great similarities with the natural substrate TTP (deoxythymidine triphosphate), the difference between the substrates being that IUdRTP has an iodine atom in the five-position on the nucleotide base, while TTP has a methyl group (whose van der Waal radius only is a little smaller).

$^{125}I$-IUdRTP may, for example, be prepared by treating $^{125}I$-iodine-deoxyuridine (IUdR) with Herpes simplex enzymes and subsequent chromatographic separation of the products obtained, viz. $^{125}I$-iodine-deoxyuridine-monophosphate (IUdRMP) and $^{125}I$-IUdRTP. $^{125}I$-IUdRTP as well as substrate analogues thereof will be described in more detail further on.

For measurements directly on serum specimens and the like containing substrate degrading enzymes, the substrate may be protected by the addition of one or more trinucleotides which do not take part in the polymerization reaction but will engage the degrading enzyme in question. For inter alia the above mentioned substrate $^{125}I$-IUdRTP, cytidine triphosphate (CTP) has been found to be an excellent protective factor. Other protective factors that may be contemplated are, e.g., UTP, IUTP, 5-MeUTP.

The modified variation of the inventive method including an initial "fish-step" may be used directly on clinical specimens, various isolation materials, lymphocytes, supernatants, etc., in contrast to the prior art

methods, which for virus polymerases have been limited to pelleted viral materials or purified samples. For example, an assay may be performed directly on lymphocyte extracts without a preceding culture or purification thereof.

A presently interesting application of the invention as far as activity determination for reverse transcriptase is concerned, is the determination of replication activity of HIV, the virus which eventually causes AIDS. The amount of reverse transcriptase from HIV in a patient specimen is a measure of this activity, and an accurate determination thereof is of great value for an analysis of the relationship of the activity with the development of the disease and a follow-up of the result of therapy. In this context the possibility of being able to determine the individual patient's enzyme sensitivity to various nucleotide analogues used as therapeuticals appears to be extremely interesting.

Concerning HIV the invention may advantageously also be used when measuring antibodies that block HIV reverse transcriptase activity; it has been found that the level of antibodies against reverse transcriptase in sera is related to the stage of the disease (see e.g. R. Schatterjee et al., J. Clin. Immun., Vol. 7, No. 3, 1987), and a measurement of the antibody level is therefore interesting from a prognostic point of view. Such an antibody measurement, which previously required purification of IgG-fractions from the serum, may with the inventive method be performed directly on the clinical serum specimen. Thus, a defined quantity of HIV reverse transcriptase is incubated with different serum dilutions, the residual amount of non-blocked enzyme then being determined by the method of the invention.

Essential for the possibility of a direct assay with the inventive method is, firstly, the increased sensitivity which permits the measurement of low enzyme amounts, causing high antibody titres to be obtained, and, secondly, that, as mentioned above, non-enzyme disturbing nucleotide(s), such as cytidine triphosphate (CTP), may be added to engage substrate degrading enzymes in sera. The latter will permit long duration assays with serum present. These criteria for increased sensitivity in measurements of HIV reverse transcriptase are also essential for direct DNA polymerase measurements in sera from healthy individuals and from those suffering from various kinds of diseases.

Due to the improved sensitivity of the method of the invention in comparison with the prior art determination methods the invention will also be applicable to polymerase determinations which have hitherto not been possible.

For example, in W. Sibrowski et al., Z. Gastroenterologie 1987, 25, 673-676, there is suggested the possibility of detecting NANB hepatite by determining the activity of the reverse transcriptase of the causal retrovirus.

Furthermore, for example, reference levels of DNA polymerase may be measured directly in sera from healthy individuals, which in combination with enhanced levels in various diseases will provide a new clinical parameter for the judgement of diseases.

The polymerase determination method according to the invention is, of course, not limited to specimens from human beings, but it may just as well be applied to animal specimens, e.g. for determining leukoviral activity.

The application of the method of the invention to quantitative determination of HIV reverse transcriptase may, for example, be performed in the following way:

An immobilized template functional for viral reverse transcriptase (e.g. poly(rA)-polystyrene beads) is preincubated directly with a crude specimen (e.g. a supernatant, cell extract, serum). After careful washing (e.g. distilled water) a suitable reaction mixture is added, comprising a primer (e.g. oligo$(dT)_{10-18}$), a labeled substrate (e.g. $^{125}$I-IUdRTP) and other necessary components (including $Mg^{2+}$). After incubation and wash (distilled water) the radioactivity incorporated into the template is measured by a gamma-counter. The measurement value obtained is directly related to the activity of reverse transcriptase in the specimen.

While carrying out the inventive method with a gel form or particulate template in conventional test tubes will offer considerable advantages in comparison with current methods, the technical procedure may be further simplified, for example, by binding the template to a single handable macrosphere or ball or by carrying out the assay in a minicolumn system.

As mentioned above preferred radiolabeled substrates for the purposes of the invention are constituted by $^{125}$I-IUdRTP and substrate analogues thereof. By substrate analogues of $^{125}$I-IUdRTP are meant $^{125}$I-labeled modified nucleoside triphosphates having substantially the same substrate function as $^{125}$I-IUdRTP. $^{125}$I-IUdRTP has the following structural formula

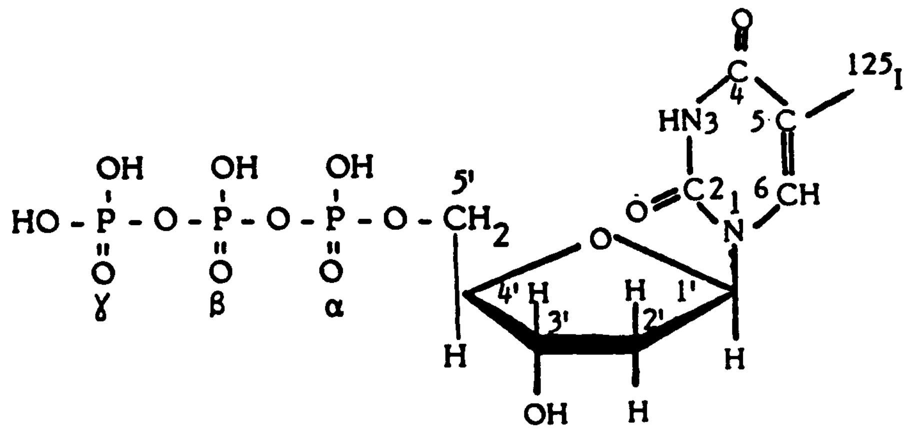

As examples of contemplated substrate analogues may thus be mentioned those where one or more of the hydrogens in the positions 3, 6, 2', 3', 4' and 5' are replaced by fluorine and/or one or more of the hydrogens in 4'- and 5'-position are replaced by $CH_3$, $CH_2F$, $CHF_2$, $CF_3$, iodine or bromine and/or one or more of the oxygen atoms present in the molecule are replaced by sulphur atoms, particularly the oxygen atoms of the $\alpha$-phosphate group. Alternatively, or in combination with the above mentioned modifications, $^{125}$I-atoms may be in the 4'- or 5'-position, the 5'-position optionally being substituted with $CH_3$, $CHF_2$, $CF_3$, iodine or bromine.

The use of $^{125}$I-IUdRTP and its substrate analogues also offer a considerable improvement in determination methods based upon free template in solution. The use of $^{125}$I-IUdRTP and its substrate analogues for determining polymerase activity in general is the object of our copending PCT-application entitled "Substrate for polymerase activity determination", the disclosure of which is incorporated by reference herein.

The present invention also provides, in a second aspect thereof, a kit for determining polymerase activity. Such a kit comprises a carrier-bound template, and at least one gamma-radiating radiolabeled nucleoside triphosphate complementary to the template, preferably $^{125}$I-IUdRTP or a substrate analogue thereof. In case a primer is required, such may be included as a separate item in the kit, or the template may be provided in a primed form. A kit for the determination of antibodies against HIV-RT as outlined above will additionally comprise a given quantity of HIV-RT, preferably lyophilized. The above kits suitably also comprise positive and/or negative controls and wash fluid.

Hereinafter the invention will be described in more detail with respect to the preferred substrate $^{125}$I-IUdRTP, the preparation of some immobilized templates, studies of parameters in specific embodiments of the inventive method as well as some special working examples.

The following abbreviations and tradenames are used:

| | |
|---|---|
| IUdRTP | = 5-iodine-2'-deoxyuridine triphosphate |
| IUdRPM | = 5-iodine-2' -deoxyuridine monophosphate |
| IUdR | = 5-iodine-2'-deoxyuridine |
| prA | = poly(rA) = polyadenylic acid |
| prG | = poly(rG) = polyguanylic acid |
| ATP | = adenosine triphosphate |
| DTE | = dithioerythritol |
| DEAE-cellulose | = diethylaminoethyl-cellulose |
| RT | = reverse transcriptase |
| EGTA | = ethylene glycol bis-($\beta$-aminoethylether)-N,N'-tetraacetic acid |
| NP 40 | = Nonidet P40 |
| HEPES | = N-2-hydroxyethylpiperazine-N'-Z-ethanesulphonic acid |
| odT | = oligodeoxythymidylic acid |
| TTP | = thymidine triphosphate |
| CTP | = cytidine triphosphate |
| TCA | = trichloroacetic acid |
| BSA | = bovine serum albumin |
| AMV | = avian myeloic leukemia |
| HIV | = human immunodeficiency virus |
| PBL | = peripheral blood lymphocytes |

| | |
|---|---|
| Sepharose® | = a trademark for an agarose gel marketed by Pharmacia AB, Sweden |
| Ficoll® | = a trademark for a copolymer of sucrose and epichlorohydrin marketed by Pharmacia AB, Sweden |
| Triton X-100 | = polyethyleneglycol-p-isooctylphenylether |
| TLC | = thin layer chromatography |

Reference will be made to the accompanying drawings wherein:

Fig. 1A illustrates time dependence of substrate turn-over with AMV-RT using primer-modified Sepharose® -prA gel as immobilized template.

Fig. 1B illustrates time dependence of substrate turn-over with partially purified HIV-RT using primer-modified Sepharose® -prA gel as immobilized template.

Fig. 1C illustrates time dependence of substrate turnover with AMV-RT using Norwegian magnetic beads as immobilized template.

Figs. 2A and 2B illustrate the relation between amount of AMV-RT and catalytic activity using prA-Sepharose® (Fig. 2A) and prA-Norwegian magnetic beads (Fig. 2B) as immobilized template.

Fig. 3A illustrates the relation between primer ($odT_{12-18}$) concentration and catalytic activity of AMV-RT using prA-Norwegian magnetic beads as immobilized template.

Fig. 3B illustrates the relation between primer ($odT_{12-18}$) concentration and catalytic activity of AMV-RT using prA coupled to Sepharose® as immobilized template.

Figs. 4A and 4B illustrate the effect on substrate turnover of concentration of immobilized template at constant template/primer ratio using primer-modified Sepharose® (Fig. 4A) and prA-Norwegian magnetic beads (Fig. 4B) as immobilized template.

Fig. 5A illustrates recovery of AMV-RT from cultured PBL extracts as a function of time. Broken line indicates direct assay and unbroken line indicates modified method with initial "fish" step. o-o represents reference enzyme without PBL extract, and ∇-∇, +-+ and △-△, respectively, represent different lymphocyte extracts plus reference enzyme.

Fig. 5B illustrates recovery in "fish" assay of AMV-RT mixed into serum as a function of time. x-x represents reference enzyme without serum and o-o different sera mixed with reference enzyme.

Fig. 6A illustrates recovery of HIV-RT from infected PBL culture supernatants as a function of time. o-o represents undiluted sample and x-x 1/5 dilution of sample. Broken line is direct assay, unbroken line "fish" assay.

Fig. 6B illustrates recovery of HIV-RT from infected PBL extracts as a function of time. o-o represents 1/5 dilution and x-x 1/25 dilution of sample. Broken line is direct assay, unbroken line "fish" assay.

Fig. 7 illustrates the influence of PBL extract on RT assay. o-o represents direct assay with immobilized template, and ●-● represents assay with initial "fish" step; --- indicates reference enzyme level in the absence of PBL.

Fig. 8 illustrates the effect of different washing procedures on the recovery of AMV-RT from extracts from $1.5 \cdot 10^5$ lymphocytes using immobilized template and initial "fish" step. x-x and △-△ represent different washing procedures, and o-o represents control recovered from buffer.

Fig. 9 illustrates the influence of template destroying factors in PBL extracts. The prA gel was preincubated with extracts from: o-o $1.9 \cdot 10^4$ PBL cells; ●-● $7.8 \cdot 10^4$ PBL cells; △-△ $3.1 \cdot 10^6$ PBL cells. --- indicates preincubation in buffer (reference level). The capacity of the gel to function as template in RT assay was then determined with AMV-RT.

Fig. 10 illustrates the effect of different concentrations of reducing agent (DTE) on RNA-DNA hybrid destroying enzymes in PBL extracts. ■-■ represents measurement on gel and □-□ measurement in supernatant.

Fig. 11 illustrates the inhibiting effect of prG on the activity of RNA-DNA hybrid destroying enzymes in PBL extracts. ■-■ represents measurement on gel and □-□ measurement in supernatant.

Figs. 12A and 12B illustrate the effect of different concentrations of prG on HIV-RT detection in immobilized template assay without (Fig. 2A) and with (Fig. 2B) initial "fish" step.

Fig. 13A illustrates the RT blocking capacity of a HIV positive serum (□-□) compared with that of a HIV negative serum (o-o).

Fig. 13B illustrates the necessary amount of RT blocking HIV negative serum for 50% inhibition of HIV-RT at different HIV-RT dilutions: ●-● 1/1; x-x 1/5; ■-■ 1/25; ▲-▲ 1/125. o-o represents AMV (1/5).

Fig. 14A is a bar chart representation illustrating the result of screening sera from healthy blood donors and HIV Western blot positive sera for HIV-1 RT blocking antibodies, using RT assay with $^{125}$I-IUdRTP and immobilized template.

Fig. 14B is a bar chart representation showing the distribution of RT blocking capacity of a large cohort of sera sampled before and after HIV Western blot (WB) positivity of the patients. Stage 0 equals WB

negative; stage 2 WB positive but asymptomatic; stage 3 WB positive, only swollen lymph nodes; stage 4 WB positive, more symptoms equal ARC or AIDS.

Fig. 15A illustrates the serum degradation of $^{125}$I-IUdRTP with time at different concentrations of CTP.

Fig. 15B illustrates the effect of different concentrations of CTP on the activity of serum DNA polymerase.

## PREPARATION OF $^{125}$I-IUdRTP (SUBSTRATE)

A. Enzyme production

Herpes simplex virus type 1, strain C42, was cultured on BHK-C13S cells, as described by Gronowitz S., Kallander C., Infec. Immun. 29: 425-434 (1980). 16-24 hours after the viral infection the cells were pelleted in a centrifuge, and the cells were washed in physiological saline and thereafter resuspended in 10 ml of a buffer consisting of HEPES, 25 mM; $MgSO_4$, 10 mM; ATP, 4 mM; DTE, 10 mM; and glycerol, 25%; pH 7.4. The cells were then sonicated for 2x30 seconds. After a subsequent centrifugation of the cells at 250,000 x g for 120 min., the supernatant was diluted 1/8 with distilled water and frozen in small portions at -70°C.

B. Synthesis of $^{125}$I-IUdRMP and $^{125}$I-IUdRTP

For the synthesis of $^{125}$I-IUdRMP and $^{125}$I-IUdRTP a reaction mixture with the following components was prepared: HEPES, 89.4 mM; $MgCl_2$, 8.1 mM; KCl, 17.7 mM; NaF, 2.1 mM; ATP, 4 mM; and DTE, 5 mM; pH 7.4. In the standard procedure the carrier solution (water:ethanol) of 2-3 mCi $^{125}$I-IUdR (2000 Ci/mMole) was evaporated. The remaining $^{125}$I-IUdR was redissolved in 500 µl of the reaction mixture and incubated at 37°C with 50 µl of the enzyme preparation obtained in step A above for the desired period of time, usually 90 to 120 minutes. After the incubation the reaction was stopped by keeping the test tube in boiling water for 5 minutes.

C. Purification

The chromatography system consisted of a standard glass column of 10 mm inner diameter packed with a 3 ml bed of DEAE cellulose gel (Whatman). The column was connected to a peristaltic pump, P-3 (Pharmacia AB, Sweden), allowing both direct and reversed flow. The flow used was 0.5-0.75 ml per min., and the absorbance ($OD_{255}$) was monitored continuously with a Single Path Monitor UV-1 connected to a Single-channel Recorder REC-481 (Pharmacia AB, Sweden).

The radioactivity in the eluent was monitored by a simple device consisting of a modified portable monitor for the detection of gamma radiation (scintillation meter type 5.40, Mini-instruments Ltd., Burnham on Crouch, England). The NaI-crystal was placed in front of the tube leading the eluent from the column. A single channel pen recorder was connected in parallel to the speaker of the monitor.

Prior to each run, the column was washed with 1M HCl and equilibrated with water. The sample was loaded on top of the column by use of the pump, and unbound material was eluted with three column volumes of distilled water. IUdR not utilized in the reaction was eluted with 10 ml of ethanol (95%). The flow of the column was reversed and material which had not entered the gel or been adsorbed was washed out with water. IUdRMP was specifically eluted from the column with 10 mM ammonium acetate, pH 5, which greatly affected the charge of the monophosphate group and decreased the binding of the IUdRMP to the DEAE cellulose. IUdRTP was finally eluted with 25 mM $MgCl_2$. The identity and purity of the product was determined by TLC.

The collected peaks were stored at +4°C after addition of 0.02% merthiolate as preservative. This IUdRTP preparation was used in the following experiments and assays without further treatment.

## PRODUCTION OF IMMOBILIZED TEMPLATES

prA coupled to magnetic beads

High molecular weight prA (Pharmacia AB, Sweden) was coupled to tosylated magnetic Dynabeads M 450 (Dynal AS, Oslo, Norway) in the presence of an excess of prA. The coupling procedure, and processing of the beads were in concordance with the manufacturer's description for coupling of IgG, except that the IgG was substituted by prA (final concentration 150 µg/ml).

prA coupled to polystyrene beads of alkylamine type

Polystyrene beads (diameter 1/4") of alkylamine type (Pierce) were activated with succinic anhydride, and high molecular weight prA (Pharmacia AB, Sweden) was coupled to the beads by the use of ethyl-3-(3-dimethylaminopropyl)-carbodiimide according to standard procedures.

prA coupled to Sepharose® (prA gel)

Poly-A-Sepharose® (Pharmacia AB, Sweden), a commercial product of low molecular prA coupled to Sepharose®.

prA coupled to polycarbonate beads

High molecular weight prA (Pharmacia AB, Sweden) was covalently coupled to polycarbonate beads by the use of ethyl-3-(3-diethylaminopropyl)-carbodiimide in accordance with standard procedures to link the prA amino group to the carboxyl group of the bead.

Activated DNA coupled to Sepharose®

A) Coupling to activated CH-Sepharose®

Activated (deoxyribonuclease treated) calf thymus DNA (Pharmacia AB, Sweden) was coupled to activated CH-Sepharose® (Pharmacia AB, Sweden). The manufacturer's coupling procedure and recommended incubation times were used. To 0.5 g Sepharose® were taken 1.5 mg of DNA. The product was kept in 50 mM of Hepes buffer, pH 8.0, with 0.05% merthiolate as preservative.

B) Coupling to CNBr-Sepharose®

Activated DNA as above was coupled to cyanogen bromide activated Sepharose® (Pharmacia AB, Sweden), in accordance with the manufacturer's description with the following modifications; coupling buffer: 10 mM $KH_2PO_4$; blocking agent: ethanol amine; wash: 200 mM KCl alternating with tris-buffer, pH 8.0. The product was kept in the tris-buffer with 0.05% merthiolate. (To 0.5 g of Sepharose® were taken 1.5 g of DNA).

Non-activated DNA coupled to a solid phase

Previous studies have demonstrated that single-stranded DNA easily may be coupled to a solid phase, while the yield in the attempts to couple double-stranded DNA has been low. This problem has previously been circumvented by establishing single-stranded regions terminally in double-stranded DNA. To obtain a good yield of coupled double-stranded DNA the temperature dependence of the DNA-hybridization was utilized herein, i.e. the chemical coupling to the solid matrix was performed at a high temperature of 90-100 °C since single-strand conditions will then prevail. The temperature was then lowered either quickly or slowly depending on how well the base pairing was to take place, i.e., whether many nicks and a great disorder was desired or not. For the purpose of increasing the amount of nicks and single-strand regions also a solid matrix with coupled DNA as above was heated to 90-100 °C, and at this temperature sufficiently strongly nicked homologous DNA, i.e. short pieces of similar DNA, alternatively homologous RNA, was added, all for the purpose of increasing the utility of the coupled DNA as a substrate when quantitating DNA polymerizing enzymes.

A) Primary production of immobilized template

DNA (2.5 mg/ml) was dissolved in 0.01M $KH_2PO_4$ while heating to 90-95 °C. CNBr-4B gel (Pharmacia AB, Sweden), swollen and washed in accordance with the manufaturer's instructions, was then added dropwise with stirring (1 g dry weight/100 ml of DNA solution). The coupling reaction was allowed to proceed for 4 hours to over-night with continued stirring and at 90-95 °C, whereupon the gel was allowed to sediment and the supernatant was removed. 100 ml of ethanol amine were then added and the mixture was stirred for 2 hours for inactivating any CNBr groups not consumed. Finally the gel was washed in a glass filter funnel with a 90-100 °C warm solution of 1) 200 mM KCl in 5 mM tris-HCl, pH 8.0, and 2) only 5 mM

tris-HCl. Thereafter the gel was suspended in 5 mM tris-HCl, pH 8, and was then allowed to cool, or the whole of it was placed in a bath at 90 °C, the heating of which was then switched off to permit the gel to cool slowly. The completed matrix was then kept in the tris-HCl buffer after the addition of sodium azide.

B) Further activation of immobilized DNA template

The utility of the immobilized template in a DNA polymerase assay may be amplified by introducing several nicks or primer sites. The conditions for an optimum template varies for different types of DNA polymerizing enzymes. A fundamental description of template activation will be given hereinafter.

Immobilized template as above was heated to 90-100 °C, and the desired primer, e.g. various degrees of DNAse-degenerated homologous DNA, was then added. The temperature was subsequently lowered to obtain base pairing, and the gel was then washed under stringent conditions with salt and tris-HCl buffer to remove partially bound DNA. In analogous manner the standard template may be improved by high temperature addition of homologous RNA primer or, alternatively, synthetic DNA or RNA primer.

## RT-ACTIVITY DETERMINATION METHODS

RT-activity determination with free template

The assay procedure is a modification of previously published procedures, e.g. in A.D. Hoffmann et al, Virology 147, 326-335 (1985).

The reaction mixture consisted of (final concentrations): tris-HCl, 10 mM, pH 8.0; $MgCl_2$, 4 mM; BSA, 0.5 mg/ml; $NH_4Cl$, 100 mM; NP 40, 0.37%; EGTA, 0.25 mM; prA, 0.625 µg/ml; $odT_{12-18}$, $8x10^{-6}$ mg/ml; $^3$H-TTP, $6x10^{-7}$ M. $^{125}$I-IUdRTP was used instead of $^3$H-TTP at about $5x10^{-8}$ M (specific activity 220 Ci/mMole), unless otherwise specified. The assay volume, including 10% specimen, was 250 µl. The reaction mixture and specimen (containing polymerase) were mixed in a test tube and incubated for the desired time period with slow shaking at 30 °C. The reaction was stopped by pipetting aliquots, usually 40 µl, onto 3 $cm^2$ small glass fibre filters, the RNA/DNA-hybrid was precipitated by TCA, and after washing the radioactivity of the precipitate was measured with a gamma-counter (Packard Autogamma 200), the radioactivity representing the incorporation of labeled $^{125}$I-IUdRTP into the template.

RT-activity determination according to the invention - standard procedure

The final concentration of the components in the assays were, unless otherwise specified: tris-HCl buffer, pH 8, 10 mM; KCl, 75 mM; $MgCl_2$, 4 mM; EGTA, 0.19 mM; DTE, 10 mM; BSA, 0.5 mg/ml; NP-40, 0.38%; $odT_{10-18}$, 1.0 µg/ml for short templates and 0.05 µg/ml for long templates; IUdRTP $5.0 \cdot 10^{-8}$ mM and the use of 0.1-1.0 µCi $^{125}$I-IUdRTP per tube; 50 µl/tube of template immobilized on magnetic beads ($10^7$/ml) or to gel (5 g/40 ml), or on a macroball (of Pierce type); 50 µl of sample for enzyme analysis. The total assay volume was 500 µl unless otherwise indicated. After mixing the components (stock solutions), adding immobilized template and enzyme sample the test tubes were incubated at 30-35 °C, and the reaction was stopped after the desired time by washing the solid matrix five times in 2.5 ml of distilled water with 0.5% Triton X-100. The $^{125}$I-IUdRMP incorporated onto the solid matrix was determined in a gamma-counter. Normally the analyses were performed with triplicate samples, and the reaction was terminated after about 1-2 hours for the first test tube, after 3-4 hours for the second test tube, and the last tube was incubated overnight in order to control the linearity of the enzyme activity with time.

RT-activity determination according to the invention - modified procedure with enzyme purification

In order to remove disturbing factors and to concentrate RT 0.025-1.0 ml of cell culture fluid, lymphocyte extract, or serum was first incubated with 0.050 ml solid template for 20-30 minutes at 30 °C or 8 °C. The immobilized template was then washed four times (2.5 ml) with distilled water alternatively twice with 3 ml of Tris-HCl buffer, pH 7.7, whereupon components for RT-activity determination according to the invention were added to the above specified final concentrations, and the RT-activity was determined by the above described standard procedure.

DNA polymerase determination with immobilized template

The reaction mixture consisted of tris-HCl, 10 mM, pH 8; $MgCl_2$, 4 mM; bovine serum albumin, 0.5 g/l; 100 mM each of deoxyadenosine triphosphate, deoxyguanosine triphosphate and deoxycytidine triphosphate; cytidine triphosphate, 0.5 mM; non-radioactive iodine-deoxyuridine triphosphate, 50 nM; calf thymus DNA immobilized on CNBr-4B gel (Pharmacia AB, Sweden), 50 µl gel suspension; dithioerythritol, 5 mM. $^{125}$I-IUdRTP, 1-5 nM (2-10 mCi/l). The final volume, including 10 volume-% sample was 500 µl.

After rinsing off the substrate not consumed from the template by washing 5 times with 10 µM $NaH_2PO_4$ in distilled water the amount of radioactive nucleotide incorporated into the DNA was determined by measuring the gel in a gamma-counter.

**STUDY OF RT-ACTIVITY DETERMINATION PROCEDURES**

Hereinafter experiments performed with the standard and, respectively, modified RT-activity determination method with regard to various parameters will be described.

RT and clinical specimens used

Purified AMV-RT (Pharmacia AB, Sweden). Clinical specimens were simulated by mixing this enzyme and the specimen investigated.

Gene-cloned HIV-RT derived from bacteria (from Prof. Bror Strandberg, BMC, Uppsala, Sweden).

Lysate from HIV-infected lymphocyte cultures treated with Triton X100 (final conc. 1.3%) (obtained from the National Swedish Bacteriological Laboratory, Stockholm, Sweden).

Supernatants from virus isolation attempts (obtained from the National Swedish Bacteriological Laboratory, Stockholm, Sweden).

Whole blood cell extracts were obtained from Rikshospitalet, Copenhagen, Denmark and prepared by washing the cells 6-fold in a physiological solution, whereafter the cells were resuspended to original concentration in Triton X-100 and frozen at -70 °C before use in assay.

Efficiency of immobilized template

In accordance with the above described standard procedure for RT-activity determination according to the invention, analyses of various amounts of AMV-RT and gene-cloned HIV-RT, respectively, were performed with the previously described immobilized templates for determining the capacity thereof. The results are shown in Figures 1-4 on the accompanying drawings.

Fig. 1A, B shows the time dependence of substrate turnover with AMV-RT (Fig. 1A) and partially purified HIV-RT (Fig. 1B) with primer-modified Sepharose®-prA gel as immobilized template. The available $^{125}$I-IUdRTP was about 230,000 cpm per tube, and the 1/3200 dilution of AMV-RT corresponds to 0.28 units of enzyme. From Fig. 1A it appears that a linear incorporation with time was achieved with low enzyme amounts while most substrate was incorporated already after a short time with an excess of AMV enzyme. The results shown in Fig. 1A also indicate that enough template as compared to IUdRTP substrate was present on the matrix.

Fig. 1C illustrates the time dependence of substrate turnover with AMV-RT using prA bound to Norwegian magnetic beads as immobilized template. The available $^{125}$I-IUdRTP was approximately 103,000 cpm per tube, and the 1/1600 dilution of AMV-RT corresponds to 0.56 units of enzyme.

Fig. 2A, B illustrates the relation between AMV-RT amount and catalytic activity when using prA-Sepharose® (Fig. 2A) or prA-Norwegian magnetic beads (Fig. 2B) as immobilized template. The available $^{125}$I-IUdRTP was approximately 230,000 and 103,000 cpm per tube for Fig. A and Fig. B, respectively, and the 1/1600 dilution of AMV-RT corresponds to 0.56 units of enzyme. It will appear from these figures that the substrate turnover was proportional to the enzyme amount over a wide range as long as an excess of substrate was present.

Fig. 3A shows the relation between the concentration of primer ($odT_{12-18}$) and catalytic activity of AMV-RT when using prA-Norwegian magnetic beads. 3.5 units of AMV-RT were used per tube. The 1/100 dilution of primer corresponds to 0.5 µg per tube. As appears from the figure no enzyme reaction was obtained in the absence of primer.

Fig. 3B shows a corresponding experiment performed with the use of AMV-RT (7.5 units/tube) and prA coupled to Sepharose®.

Fig. 4A, B shows the effect of concentration of immobilized template at constant template/primer ratio when using primer-modified prA-Sepharose® (Fig. 4A) or prA-Norwegian magnetic beads (Fig. 4B). The available $^{125}$I-IUdRTP was approximately 600,000 cpm per tube, and approximately 2.5 units of AMV-RT were used per tube. The 1/1 amount of modified prA-Sepharose® corresponds to 50 $\mu$l of gel per tube, while the 1/1 amount of prA-Norwegian magnetic beads corresponds to $4x10^7$ beads per tube. As appears from Fig. 4B the template/primer amount was rate-limiting with incorporation of low substrate amounts at all tested concentrations of Norwegian beads, while Fig. 4A shows that a saturated system was achieved at about 25 $\mu$l of modified prA-gel per assay tube.

Effect of crude clinical specimens and lymphocyte extracts in the standard method of the invention

To demonstrate the difficulties in determining RT-activity directly upon crude clinical specimens and lymphocyte extracts with the standard method of the invention, and thus also with the previously known methods based upon the use of a free template, the following experiments were performed on extracts and supernatants from PBL cultures.

The experiments were carried out by mixing the samples with a suitable amount of purified AMV-RT, and then analysing the RT-activity with the previously described standard method for RT-activity determination according to the invention using prA-Sepharose® template. The results are shown in Table 1 which gives the recovered percentage of AMV-RT activity in relation to assay time.

Table 1

| Type of sample | % sample in assay mixture | Recovery of AMV-RT activity (%) after | |
|---|---|---|---|
| | | 1-2 h | over-night incubation |
| Control | 0 | 100 | 100 |
| Medium from PBL cultures | | | |
| c803 | 50 | 89.9 | 133.0 |
| c808 | 50 | 115.2 | 110.0 |
| c809 | 50 | 113.0 | 137.8 |
| c801 | 50 | 88.9 | 75.3 |
| c748 | 50 | 35.2 | 12.3 |
| Extract from PBL cultures | | | |
| c803 | 50 | 44.4 | 1.4 |
| c808 | 50 | 24.7 | 0.2 |
| c809 | 50 | 45.0 | 4.5 |
| c801 | 50 | 17.0 | 0.0 |
| c748 | 50 | 8.4 | 0.5 |
| Human serum | | | |
| Tk7485 | 30 | 0.0 | 1.6 |
| Tk9232 | 30 | 8 | 14.5 |
| Tk12310 | 30 | 12.9 | 13.5 |
| p1 | 10 | 79.7 | 99 |
| p1 | 50 | n.d. | 59.4 |
| p2 | 10 | 68.6 | 79.4 |
| p2 | 50 | 19.2 | 97.9 |
| n.d. = not determined | | | |

As appears from Table 1 above the PBL extracts strongly reduced the recovery of RT-activity already after a short assay time, while the long duration assay showed that the product was broken down. In contrast only one of the five supernatants from uninfected cell cultures had a significant effect on the RT recovery.

The attempts to recover RT-activity from samples with high serum concentrations were also complicated by an unspecific background increase, probably due to protein precipitation. The experiment results show, however, that the degree of disturbance varied both with the serum concentration and with the serum origin when sera from healthy persons and persons with different diseases were compared, as exemplified for pernicious anemia (p) in Table 1. Further, the effect of serum was of competitive nature and product destruction hardly seemed to take place.

Binding of RT to template in the standard and, respectively, modified method

The ability of the immobilized template to bind RT was investigated in the following way:

AMV-RT or HIV-RT was mixed with prA-Sepharose® and incubated for 20-90 minutes at 30 °C under constant mixing, and the gel was then washed four times and the amount of bound RT determined by adding reaction mixture including primer to initiate the reaction as described above. Corresponding experiments were performed except that no washing of the gel was performed before adding the reaction mixture, i.e. equivalent to the above described standard method of the invention. The results from representative experiments with AMV-RT and gene-cloned HIV-RT are given in Table 2A below. Concerning the assay procedures in Table 2A, "direct" indicates the standard method, while "fish" indicates the modified method of the invention with an initial "fish" of RT.

Table 2A

| Expt. No. | AMV-RT (units/ tube) | Assay proce- dure | Assay time I min. | cpm I | Assay time II min. | cpm II | Assay time III min. | cpm III | Recovery of RT-activity at times I/II/III (%) | Fish time min. | Additional |
|---|---|---|---|---|---|---|---|---|---|---|---|
| 1 | 1.7 | direct | 120 | 3 580 | 1 010 | 38 284 | 1 305 | 41 432 | | | |
| | | fish | 120 | 3 452 | 1 010 | 29 008 | 1 305 | 32 640 | 96/76/128 | 20 | |
| | | fish | 120 | 3 460 | 1 010 | 43 984 | 1 305 | 53 029 | 97/115/128 | 95 | |
| 2 | 0.8 | direct | 120 | 2 284 | 225 | 6 586 | 4 200 | 46 296 | | | |
| | | fish | | (5 396) | | 5 184 | | 31 984 | (236)/79/69 | 20 | 2 wash after fish |
| | | fish | | 1 176 | | 3 588 | | 26 160 | 51/54/57 | 20 | 4 -"- |
| | | fish | | 2 056 | | 4 972 | | 29 752 | 90/75/64 | 45 | 2 -"- |
| | | fish | 120 | 1 656 | 225 | 3 788 | 4 200 | 32 532 | 72/58/70 | 45 | 4 -"- |
| 3 | 1.0 | direct | 90 | 22 396 | 240 | 49 606 | 1 320 | 214 472 | | | |
| | | fish | 90 | 30 212 | 240 | – | 1 320 | 234 276 | 135/–/104 | 45 | |
| 4 | 0.9 | direct | 90 | 23 178 | 180 | 43 598 | 1 475 | 253 760 | | | |
| | | fish | 90 | 13 584 | 180 | 31 340 | 1 475 | 184 860 | 59/72/73 | 60 | |
| 5 | 0.8 | direct | 120 | 17 664 | 240 | 28 768 | 1 360 | 100 500 | | | |
| | | fish | 120 | 14 376 | 240 | 26 132 | 1 360 | 114 612 | 81/91/114 | 30 | |
| | HIV-RT prep. | | | | | | | | | | |
| 6 | Q29 | direct | 95 | 9 770 | 150 | 22 927 | 1 175 | 48 743 | | | |
| | | fish | 95 | 18 940 | 150 | 37 267 | 1 175 | 116 247 | 194/163/238 | 45 | 4 wash 1.5 ml |
| 7 | H-RT | direct | 90 | 14 816 | 180 | 21 644 | 1 240 | 56 192 | | | |
| | | fish | 90 | 10 564 | 180 | 18 488 | 1 240 | 64 020 | 71/85/114 | 60 | 5 wash 2.5 ml |
| 8 | H-RT | direct | 90 | 1 056 | 180 | 2 936 | 1 035 | 14 940 | | | |
| | | fish | 90 | 1 016 | 180 | 1 952 | 1 035 | 5 540 | 96/66/37 | 30 | 4 wash 2.5 ml |

As appears from Table 2A above 80->100% of the AMV-RT activity was normally recovered on the gel, as compared to the reference, while even higher activity was recovered by the affinity purification or "fish" with immobilized template when using semi-purified HIV-RT. The table also shows the effect of different incubation times for the affinity purification step.

Time dependence for RT recovery from PBL supernatant, extract and serum

Fig. 5A shows the results of experiments concerning the recovery of AMV-RT activity from cultured PBL extracts as a function of time. In the figure the unbroken lines represent the modified method comprising the fish step, while the broken lines relate to the standard method with direct assay. The experiment was performed with three different PBL extracts (C803, C808 and C809, respectively). As reference was used the corresponding amount of AMV-RT (1.8 units per tube) not mixed with PBL extract.

Fig. 5B shows the corresponding result according to "fish" precedure with AMV-RT mixed into serum. The experiment was performed with 6 different serum sample mixtures consisting of 200 µl of undiluted serum with 10 µl of enzyme added (0.9 unit per tube). The recovery of AMV-RT from buffer was used as a reference.

Fig. 6A, 6B show results from experiments with fish of HIV-RT from clinical specimens, viz. from supernatants of infected PBL cultures (Fig. 6A) and when using PBL extracts (Fig. 6B). A comparison was made between the standard method with direct assay (broken lines) and the modified method with fish (unbroken lines). Modified prA-Sepharose® was used as the immobilized template.

To see whether the new technique was sensitive enough to detect RT activity in blood cell extracts from HIV infected persons, 5 samples and controls were analysed using direct and fish procedure with $^{125}$I-IUdRTP and prA-Sepharose®.

The results presented in Table 2B below show that RT activity could be detected both in direct and fish assay using material from HIV infected while no activity was found for the controls. Although the highest values were found with the fish procedure, the results (Table 2B) show that disturbing factors are present as good linearity with time and sample amount was not achieved.

## Table 2B

RT activity in blood cell extracts derived from HIV infected patients and controls.

| Sample no | HIV infection (+ or -) | [a]Product recovery in direct assay: cpm time 1 | cpm time 2 | [c]linearity factor | [a]Product recovery in "fish assay": cpm time 1 | cpm time 2 | [c]linearity factor |
|---|---|---|---|---|---|---|---|
| 1 | + | 896 | 2456 | 2.74 | 880 | 716 | 0.81 |
| * ¼ | | 188 | 2140 | b | 340 | 752 | 2.21 |
| 2 | + | 372 | 2868 | 7.71 | 624 | 1600 | 2.56 |
| * ¼ | | 1616 | 2480 | 1.53 | 316 | 900 | 2.85 |
| 3 | + | 860 | 3236 | 3.76 | 2024 | 6664 | 3.00 |
| * ¼ | | 268 | 3228 | b | 696 | 2080 | 4.35 |
| 4 | + | 1296 | 5432 | 4.19 | 1728 | 5192 | 3.00 |
| * ¼ | | 136 | 2240 | b | 968 | 4208 | 4.35 |
| 5 | + | 1424 | 3924 | 2.76 | 3744 | 6872 | 1.84 |
| * ¼ | | 112 | 3200 | b | 816 | 3620 | 4.43 |
| 6 | - | 900 | 188 | b | 92 | 68 | b |
| * ¼ | | 32 | 68 | b | 16 | 104 | b |
| 6 + RT | - | 16360 | 163696 | 10.0 | 11264 | 99492 | 8.83 |
| *¼ + RT | | 23036 | 191608 | 8.32 | 14164 | 128708 | 9.08 |
| control RT | | 20940 | 191372 | 9.14 | 10596 | 108016 | 10.19 |

[a]Values corrected for background. [b] Value not reliable, too small values. [c] Linearity factor; cpm time 2 divided with cpm time 1. * ¼ The sample was diluted 1 to 4.

From the results shown above it appears that a good recovery was obtained both from PBL supernatants, indicating absence of affinity disturbing factors, and from serum, showing that the competitive agents are not bound but may be washed away. The data for the PBL extracts show that the bulk of product destroying substances is removed in the affinity step and recoveries of between 40 and 75% of the AMV-RT activity were achieved.

Since a substantially quantitative recovery is desirable and since it could not be excluded that the product destroying factors reduced the prA amount on the solid phase and in addition removed some of the RT bound to the prA template, the experiments described below were performed.

## MEASUREMENT OF PRODUCT-TEMPLATE DESTROYING FACTORS IN PBL EXTRACTS AND INVESTIGATION OF PROTECTIVE AGENTS

To measure the disturbing activities in crude specimens two different pilot systems were used.

According to the first system a crude specimen was incubated with immobilized template (prA-Sepharose®) without primer for a given time followed by a thorough wash of the template, and the function of the template was then analysed by the unmodified assay procedure of the invention.

According to the second system a $^{125}$I-labeled template hybrid was produced by long duration incubation of an excess of AMV-RT with a complete reaction solution followed by a thorough wash. The removal of product from the gel was then determined for different clinical specimens.

For the experiments a large batch of extracts of PBL isolated from normal human buffy coats was produced by Ficoll® gradient centrifugation.

### Disturbance of PBL extracts in RT activity determination

The RT assay disturbing capacity of the PBL extract was tested by performing the standard as well as the modified method of the invention on a prA gel. Serial dilutions of the PBL extract (undiluted cell extract corresponds to $10^7$ cells/ml) were incubated for 240 minutes in an assay mixture with 0.28 units of AMV-RT and 300,000 cpm $^{125}$I-IUdRTP available. The RT activity in the absence of PBL extract was measured as control. The results are shown in Figure 7. As appears from this figure the PBL extract had the capacity to disturb direct RT-measurements (the standard method) at least down to a dilution corresponding to 1.0 x $10^4$ lymphocytes/ml. Using the modified assay method including an initial "fish" procedure the disturbing effect in a 140 minutes assay could be eliminated up to a PBL concentration of 7.8 x $10^4$ lymphocytes/ml.

### Effect of different washing procedures on RT recovery

The effect of different washing procedures in the fish step on the recovery of AMV-RT activity from an extract of 1.5 x $10^5$ PBL/ml was determined by measuring the recovered activity with a 240 minutes assay. The PBL concentration was chosen to give a significant disturbance of the RT activity determination after the fish step but still leave a measurable enzymatic activity. The following two washing procedures were used: Wash five times with 6.6 times dilution in each step (procedure 1); and wash five times with 40 times dilution in each step (procedure 2). RT in buffer and wash of the prA gel according to procedure 1 was used as control. The results are shown in Fig. 8. As appears therefrom the recovered RT activity varied with the washing procedures used. The results thus show the presence of residual small amounts of disturbing factors even after five repeated washes.

### prA template disturbing factors in PBL extract

By means of the above mentioned first pilot system the presence of prA template disturbing factors in PBL extracts was determined in the following way:

A modified prA gel was preincubated 15, 30 and 70, respectively, minutes with each one of extracts from 7.8 x $10^4$ PBL/ml, extracts from 1.9 x $10^4$ PBL/ml, extracts from total buffy coat corresponding to 3.1 x $10^6$ PBL/ml, and extracts from total buffy coat corresponding to 7.8 x $10^5$ PBL/ml. Then the capacity of prA gel to function as a template in a 240 minutes assay with 0.28 units of AMV-RT was determined after incubation and thorough wash of the gel. The RT activity found with prA template incubated with buffer was used as control. The results are shown in Fig. 9. It clearly appears therefrom that the capacity of the prA gel to function as a template decreased when the preincubation time with the PBL extract increased, which indicates the presence of destroying enzymes.

### Effect of disturbing factors in PBL-extracts on RNA/DNA-hybrid produced and inhibition thereof

To investigate the effect of the disturbing enzymes in the PBL extract on the RNA-DNA reaction product the above mentioned second pilot system was used. Experiments (not presented here) showed that the labeled RNA-DNA hybrid was actually broken down as a linear function of time and the amount of PBL

extract used.

This pilot system was then used to study the activity degree of the disturbing enzymes and possible inhibitors. Thus, the effect of reducing agent (DTE) on the activity of RNA-DNA hybrid destroying enzymes was determined by incubating the $^{125}$I-labeled RNA-DNA hybrid for 240 minutes with extracts from 2 x $10^5$ PBL/ml at different DTE concentrations and subsequent measurement of residual radioactivity on gel and in liquid phase, respectively. Radiolabeled hybrid incubated in buffer was used as control. The results, shown in Fig. 10, indicate that the assay procedure should be performed in the absence of DTE.

In a corresponding manner the inhibiting effect of prG on the activity of the RNA-DNA hybrid destroying enzymes was determined by incubating the $^{125}$I-labeled RNA-DNA hybrid for 240 minutes with extracts from 2 x $10^5$ PBL/ml at different prG concentrations and with radiolabeled hybrid incubated in buffer as control. The results are shown in Fig. 11 and indicate that prG is a competitive inhibitor for the reaction of the disturbing enzymes.

Protection of prA template with prG

As is clear from the different experiments concerning disturbing factors described above, the prA template is partially destroyed during incubation with PBL extract, probably by RNAses. This may interfere both with the recovery of RT in the fish step and the reaction rate or linearity of the following activity determination. The disturbance during the RT activity determination might also reflect the action of the same enzyme to destroy or release the reaction product, i.e. the RNA-DNA hybrid. By the fish step in the modified RT determination method according to the invention obviously most of the product destroying enzymes are eliminated, but an agent protecting the RNA-DNA hybrid would be useful to reduce the washing procedure. An essential problem is therefore to protect the prA template during the fish step. This could be done by introducing either selective physical or chemical conditions. Both the addition of direct RNAse inhibitors and the omission of substances essential for RNAse activity may thus be contemplated. Provided that the same enzyme is responsible for the destruction of prA template and RNA-DNA hybrid, the fishing step in the RT determination procedure should, as already mentioned above, preferably be performed in the absence of DTE. Further, as is also clear from the information furnished above, prG may thus be used to protect the RNA-DNA hybrid after the fishing step in the subsequent activity determination.

Since HIV-RT is previously known to have a comparatively low affinity for prG, the latter could be a possible protective agent for the immobilized prA template. To determine the effect of different concentrations of prG on the different steps of the RT activity assay, a 75 minutes assay of the activity of purified gene-cloned HIV-RT was performed, (i) with the standard method with a direct activity determination on odT-prA gel in the presence of different concentrations of prG, and (ii) by the modified method with a preceding "fish" of HIV-RT from solutions containing different amounts of prG followed by a thorough wash of the gel, and then determination of the bound enzyme activity in absence of prG. The results for the direct enzyme activity determination are shown in Fig. 12A, and for the determination with a separate fishing step in Fig. 12B. From the results it is clear that prG substantially does not disturb the RT activity during the assay but interferes with the binding of RT to prA in the fish procedure. Herefrom it may thus be concluded that prG may be useful to protect the RNA-DNA hybrid after fish, i.e. to eliminate the residual RNAse activity.

Determination of HIV-RT blocking antibodies

The presence of HIV-RT blocking antibodies in serum samples was determined in the following way. Different dilutions of sera from a HIV-infected and, respectively, a healthy person were incubated at 37°C for 75 minutes with HIV-RT or AMV-RT for control. The residual RT activity was then determined, (i) with immobilized template and a fishing step, and (ii) with a free template in solution, CTP, however, being added as substrate protection and with 0.5 mm spermine present. The results are shown in Fig. 13A, B.

Fig. 13A thus shows the enzyme blocking capacity of a serum from a HIV positive individual compared with a serum from a HIV-negative person using a HIV-RT dilution of 1/10. The serum amount required for 50% inhibition of HIV-RT corresponds to 18 ng of IgG. Fig. 13B illustrates the relationship between the necessary amount of serum for 50% RT inhibition and the amount of RT in the assay. From the figure it is also clear that the HIV-positive serum does not block AMV-RT.

These results demonstrate that the determination of RT activity according to the present invention may be performed directly on serum due to the high sensitivity of the system. This high sensitivity is largely due to the use of $^{125}$I-IUdRTP as substrate and also upon the possibility of protecting the substrate with CTP in a long duration assay. The high sensitivity obtained, which may be exemplified by the fact that only 10 ng

of IgG were required in the above described assay compared with 300 ng for the strongest HIV serum according to Chatterjee R. et al., J. Clin. Immunol. vol. 7, No. 3, 1987, of course, means that a wider range of antibody titres may be obtained.

A similar assay as used above, but with prA coupled to polycarbonate beads as immobilized template, was used for screening large amounts of serum samples for HIV-1 RT blocking antibodies. A collection of 897 sera, sampled at different times before and after HIV-1 infection, and as controls sera from 200 healthy blood donors were investigated. Figure 14A shows that the specificity of RT blocking antibodies was extremely good, none among the blood donors was positive, while virtually all of the HIV-Western blot positive sera were HIV-RT antibody positive indicating the high sensitivity in the assay.

Figure 14B illustrates further the sensitivity of the above RT antibody test. The values from the HIV infected patients are distributed according to the stage of the disease. As appears from the figure almost all patients in stages 2-4 were RT antibody positive and some patients were positive already in stage 0 before being HIV positive according to Western blot analysis.

## STUDY OF DNA POLYMERASE ACTIVITY DETERMINATION

### Determination of activity for Klenow's enzyme

By means of the above described procedure for DNA polymerase determination with immobilized template, the activity for Klenow's enzyme on different gel templates was measured. In the assay a gel bound template produced as described above was used, (i) as such ("unprimed") and (ii) activated ("primed") by boiling 1 ml of the gel, in the presence or absence of spermidine (2 mM), with 0.4 ml of DNAse-treated DNA and wash with a high salt concentration prior to use. The DNAse-treated DNA (primer) was obtained by "nicking" 40 ml of DNA solution containing 5 mg of DNA/ml with 125 ng/ml DNAse (final concentration) at 37°C for 4½ hours; the DNAse was then inactivated by heating at 70°C for 20 min., whereupon the DNA was precipitated with ethanol and KCl, washed and rediluted to 8 ml. In the determination 2 x $10^{-3}$ and, respectively, 2 x $10^{-2}$ units of Klenow's enzyme were used. The results are presented in the following Table 3.

Table 3

| Template | Measured activity (cpm) | | | |
|---|---|---|---|---|
| | 2x$10^{-3}$ unit of Klenow | | 2x$10^{-2}$ unit of Klenow | |
| | after 45 min. | after 1005 min. | after 45 min. | after 1005 min. |
| primed | 2 810 | 66 384 | 30 540 | *199 392 |
| primed + spermidine | 1 875 | 47 796 | 20 115 | 143 644 |
| unprimed | 385 | 7 332 | 1 575 | 31 380 |
| background | 295 | 264 | 210 | 560 |

* not linear due to substrate exhaustion

From the table the excellent sensitivity improvement obtained with the activated template (about 10 times) may clearly be seen.

### Protection of $^{125}$I-IUdRTP by CTP addition

Incubation of $^{125}$I-IUdRTP with serum in the absence of template demonstrates a substrate consumption which is not due to incorporation into DNA. This may be due to the substrate being utilized by other enzymes than DNA polymerase. Thus, for example, the presence in serum of several forms of thymide triphosphate nucleotide hydrolase, an enzyme degrading TTP, has been detected. It has been found that the presence of cytidine triphosphate (CTP) in the reaction mixture eliminates the template-independent degradation of the substrate occurring in serum. The results from incubation of serum and substrate with 2 mM CTP over-night are shown in Fig. 15A and 15B. As appears from Fig. 15A less than 10% of the substrate was destroyed in the presence of 2 mM CTP, while more than 90% of the substrate was destroyed in the absence of CTP. However, high concentrations of CTP resulted in an inhibition of the serum polymerase activity, as is illustrated in Fig. 15B. With 0.5 mM CTP in the reaction mixture the best

total effect was obtained, and a considerable improvement of the linearity for the incorporation of the labeled substrate was achieved. CTP may, of course, be used in all cases where it is desired to prevent degradation of the $^{125}$I-IUdRTP substrate, provided that CTP does not disturb the desired reaction. CTP is therefore advantageously also used in the RT activity measurement, such as for HIV-RT.

## Claims

1. A method of quantitatively determining nucleic acid polymerase activity in a sample, comprising incubating the sample with a natural or completely or partially synthetic polynucleotide template and a reagent solution containing necessary substrates including at least one radiolabeled nucleoside triphosphate complementary to the template, separating the template from the substrate and measuring the radioactivity incorporated into the template, said radioactivity being substantially proportional to the polymerase activity in the sample, and wherein, when a primer is required, the latter is either hybridized to the template from the start or added during the determination, characterized in that the template is immobilized on a carrier, that said nucleoside triphosphate is labeled with a gamma-radiating isotope and that the measurement of the incorporated radioactivity is performed directly on the immobilized template without release thereof from the carrier.

2. A method according to claim 1,
characterized by first incubating the sample with the immobilized template for taking up polymerase activity thereon, and after separating the template from the sample incubating the template with the reagent solution including the radiolabeled nucleoside triphosphate for determining the polymerase activity taken up by the template.

3. A method according to claim 1 or 2, wherein the polymerase is reverse transcriptase,
characterized in that the immobilized template is a single nucleotide chain of RNA type and that the template is hybridized with a deoxyribonucleotide primer.

4. A method according to claim 1 or 2, wherein the polymerase is DNA polymerase,
characterized in that the immobilized template is a double nucleotide chain with single strand regions or a single nucleotide chain having at least one double strand region.

5. A method according to claim 1 or 2, wherein the polymerase is a RNA polymerase,
characterized in that the template, depending on the type of RNA polymerase, is either DNA with necessary primer or RNA with necessary primer.

6. A method according to any one of claims 1-5,
characterized in that said radioactive isotope is an iodine isotope, preferably $^{125}$I.

7. A method according to any one of claims 1-4 and 6,
characterized in that said nucleoside triphosphate is an iodine-2'-deoxyuridine triphosphate, preferably 5-iodine-2'-deoxyuridine triphosphate.

8. A method according to any one of claims 1-7,
characterized in that the immobilized template has been chemically modified in order not to be degraded by RNAses and DNAses, preferably by 2'-O-methylation, P-methylation or P-sulphonation.

9. A method according to any one of claims 1-8,
characterized in that the carrier on which the template is immobilized is a gel, a microsphere, a bead or a ball.

10. A method according to any one of claims 3 or 5-9,
characterized by adding one or more RNAse inhibitors, particularly polyguanylic acid, to protect the template.

11. A method according to any one of claims 1-10,
characterized by adding one or more trinucleotides which do not take part in the polymerization reaction, but which will protect the radiolabeled substrate against degrading enzymes, particularly

cytidine triphosphate.

12. Use of the method according to any one of claims 1-11 in the determination of antibodies against reverse transcriptase in a sample, particularly HIV reverse transcriptase, by incubating the sample with a defined amount of reverse transcriptase and then determining the residual, not antibody-blocked reverse transcriptase.

13. A kit for determining nucleic acid polymerase activity, comprising a carrier-bound template, at least one nucleoside triphosphate complementary to the template and labeled with a gamma-radiating isotope, and optionally a primer.

14. A kit according to claim 13,
characterized in that said radiolabeled nucleoside triphosphate is $^{125}$I-5-iodine-2'-deoxyuridine triphosphate or a substrate analogue thereof.

**Patentansprüche**

1. Verfahren zur quantitativen Bestimmung der Nucleinsäure-Polymeraseaktivität in einer Probe, umfassend das Inkubieren der Probe mit einem natürlichen oder vollständig oder teilweise synthetischen Polynucleotid-Templat und einer Reagenslösung, welche notwendige Substrate, einschließlich mindestens einem zu dem Templat komplementären, radiomarkierten Nucleosidtriphosphat, enthält, Abtrennen des Templats von dem Substrat und Messen der in das Templat eingebrachten Radioaktivität, wobei diese Radioaktivität zu der Polymeraseaktivität in der Probe im wesentlichen proportional ist, und wobei, wenn ein Primer erforderlich ist, der letztere entwedervon Beginn an mit dem Templat hybridisiert oder während der Bestimmung zugesetzt wird, **dadurch gekennzeichnet**, daß das Templat aufeinem Trägerimmobilisiert wird, daß das Nucleosidtriphosphat mit einem gamma-strahlenden Isotop markiert wird und daß die Messung der eingebrachten Radioaktivität direkt mit dem immobilisierten Templat durchgeführt wird, ohne dieses vom Träger zu lösen.

2. Verfahren nach Anspruch 1, **dadurch gekennzeichnet**, daß zuerst die Probe mit dem immobilisierten Templat inkubiert wird, um darauf Polymeraseaktivität aufzunehmen und daß nach Abtrennung des Templats von der Probe das Templat mit der Reagenslösung einschließlich dem radiomarkierten Nucleosidtriphosphat inkubiert wird, um die durch das Templat aufgenommene Polymeraseaktivität zu bestimmen.

3. Verfahren nach Anspruch 1 oder 2, wobei die Polymerase eine reverse Transkriptase ist, **dadurch gekennzeichnet**, daß das immobilisierte Templat eine einzelne Nucleotidkette vom RNA-Typ ist, und daß das Templat mit einem Desoxyribonucleotid-Primer hybridisiert wird.

4. Verfahren nach Anspruch 1 oder 2, wobei die Polymerase DNA-Polymerase ist, **dadurch gekennzeichnet**, daß das immobilisierte Templat eine Doppelnucleotidkette mit Einzelstrangbereichen oder eine Einzelnucleotidkette mit mindestens einem Doppelstrangbereich ist.

5. Verfahren nach Anspruch 1 oder 2, wobei die Polymerase eine RNA-Polymerase ist, **dadurch gekennzeichnet**, daß das Templat in Abhängigkeit des Typs der RNA-Polymerase entweder DNA mit notwendigem Primer oder RNA mit notwendigem Primer ist.

6. Verfahren nach mindestens einem der Ansprüche 1-5, **dadurch gekennzeichnet**, daß das radioaktive Isotop ein Iodisotop, vorzugsweise $^{125}$I, ist.

7. Verfahren nach mindestens einem der Ansprüche 1-4 und 6, **dadurch gekennzeichnet**, daß das Nucleosidtriphosphat ein Iod-2'-desoxyuridintriphosphat, vorzugsweise 5-Iod-2'-desoxyuridintriphosphat, ist.

8. Verfahren nach mindestens einem der Ansprüche 1-7, **dadurch gekennzeichnet**, daß das immobilisierte Templat chemisch modifiziert worden ist, um nicht durch RNAsen und DNAsen abgebaut zu werden, vorzugsweise durch 2'-O-Methylierung, P-Methylierung oder P-Sulfonierung.

9. Verfahren nach mindestens einem der Ansprüche 1-8, **dadurch gekennzeichnet**, daß der Träger, aufwelchem das Templat immobilisiert wird, ein Gel, eine Mikrokugel, eine Perle oder ein Kügelchen ist.

10. Verfahren nach mindestens einem der Ansprüche 3 oder 5-9, **dadurch gekennzeichnet**, daß ein oder mehrere RNAse-Inhibitoren, insbesondere Polyguanylsäure, zugegeben werden, um das Templat zu schützen.

11. Verfahren nach mindestens einem der Ansprüche 1 - 10, **dadurch gekennzeichnet**, daß ein oder mehrere Trinucleotide zugegeben werden, welche an der Polymerisationsreaktion nicht teilnehmen, welche jedoch das radiomarkierte Substrat gegenüber abbauenden Enzymen, insbesondere Cytidintriphosphat, schützen.

12. Verwendung des Verfahrens nach mindestens einem der Ansprüche 1-11 bei der Bestimmung von Antikörpern gegenüber reverser Transkriptase in einer Probe, insbesondere HIV reverser Transkriptase, durch Inkubieren der Probe mit einer definierten Menge reverser Transkriptase und dann Bestimmen der restlichen, nicht antikörperblockierten reversen Transkriptase.

13. Kit zur Bestimmung von Nucleinsäure-Polymeraseaktivität, umfassend ein trägergebundenes Templat, mindestens ein zu dem Templat komplementäres und mit einem gamma-strahlenden Isotop markiertes Nucleosidtriphosphat, und wahlweise einen Primer.

14. Kit nach Anspruch 13, **dadurch gekennzeichnet**, daß das radiomarkierte Nucleosidtriphosphat $^{125}$I-5-Iod-2'-desoxyuridintriphosphat oder ein Substratanalogon hiervon ist.

**Revendications**

1. Méthode pour la détermination quantitative d'une activité acide nucléique polymérase dans un échantillon, comprenant l'incubation de l'échantillon avec une matrice polynucléotidique naturelle ou complètement ou partiellement synthétique et une solution de réactifs contenant des substrats nécessaires incluant au moins un nucléoside triphosphate radiomarqué complémentaire de la matrice, la séparation de la matrice du substrat et la mesure de la radioactivité incorporée dans la matrice, cette radioactivité étant pratiquement proportionnelle à l'activité polymérase dans l'échantillon, et dans laquelle, lorsqu'une amorce est requise, cette dernière est soit hybridée à la matrice dès le début, soit ajoutée pendant la détermination, caractérisée en que la matrice est immobilisée sur un support, que ce nucléoside triphosphate est marqué avec un isotope à rayonnement gamma et que la mesure de la radioactivité incorporée est réalisée directement sur la matrice immobilisée sans libération de celle-ci du support.

2. Méthode suivant la revendication 1, caractérisée par l'incubation préalable de l'échantillon avec la matrice immobilisée pour que celle-ci prenne une activité polymérase, et après séparation de la matrice de l'échantillon, l'incubation de la matrice avec la solution de réactifs incluant le nucléoside triphosphate radiomarqué pour la détermination de l'activité polymérase prise par la matrice.

3. Méthode suivant les revendications 1 ou 2, dans laquelle la polymérase est une transcriptase inverse, caractérisée en ce que la matrice immobilisée est une chaîne nucléotidique unique de type ARN et que la matrice est hybridée avec une amorce désoxyribonucléotide.

4. Méthode suivant les revendications 1 ou 2, dans laquelle la polymérase est une ADN polymérase, caractérisée en ce que la matrice immobilisée est une chaîne nucléotidique double avec des régions à brin unique ou une chaîne nucléotidique unique ayant au moins une région double brin.

5. Méthode suivant les revendications 1 ou 2, dans laquelle la polymérase est une ARN polymérase, caractérisée en ce que la matrice, en fonction du type d'ARN polymérase, est soit un ADN avec l'amorce nécessaire, soit un ARN avec l'amorce nécessaire.

6. Méthode suivant l'une quelconque des revendications 1 à 5, caractérisée en ce que cet isotope radioisotope est un isotope de l'iode, de préférence $^{125}$I.

7. Méthode suivant l'une quelconque des revendications 1 à 4 et 6, caractérisée en ce que ce nucléoside triphosphate est un iode-2'-désoxyuridine triphosphate, de préférence le 5-iode-2'-désoxyuridine triphosphate.

8. Méthode suivant l'une quelconque des revendications 1 à 7, caractérisée en ce que la matrice immobilisée a été chimiquement modifiée pour ne pas être dégradée par des ARNases et par des ADNases, de préférence par 2'-O-méthylation, P-méthylation ou P-sulfonation.

9. Méthode suivant l'une quelconque des revendications 1 à 8, caractérisée en ce que le support sur lequel est immobilisés la matrice est un gel, une microsphère, une perle ou une bille.

10. Méthode suivant l'une quelconque des revendications 3 ou 5 à 9, caractérisée par l'addition d'un ou de plusieurs inhibiteurs d'ARNase, en particulier d'acide polyguanylique, pour protéger la matrice.

11. Méthode suivant l'une quelconque des revendications 1 à 10, caractérisée par l'addition d'un ou de plusieures trinucléotides qui ne prennent pas part à la réaction de polymérisation, mais vont protéger le substrat radiomarqué contre des enzymes de dégradation, en particulier, le cytidine triphosphate.

12. Utilisation de la méthode suivant l'une quelconque des revendications 1 à 11, dans la détermination d'anticorps dirigés contre une transcriptase inverse dans un échantillon, en particulier une transcriptase inverse de HIV, par incubation de l'échantillon avec une quantité définie de transcriptase inverse et ensuite, détermination de la transcriptase inverse résiduelle non bloquée par un anticorps.

13. Kit pour la détermination d'une activité acide nucléique polymérase, comprenant une matrice liée à un support, au moins un nucléoside triphosphate complémentaire de la matrice et marqué avec un isotope à rayonnement gamma, et éventuellement une amorce.

14. Kit suivant la revendication 13, caractérisé en ce que ce nucléoside triphosphate radiomarqué est le $^{125}$I-5-iode-2'-désoxyuridine triphosphate ou un substrat analogue de celui-ci.

FIG.1A

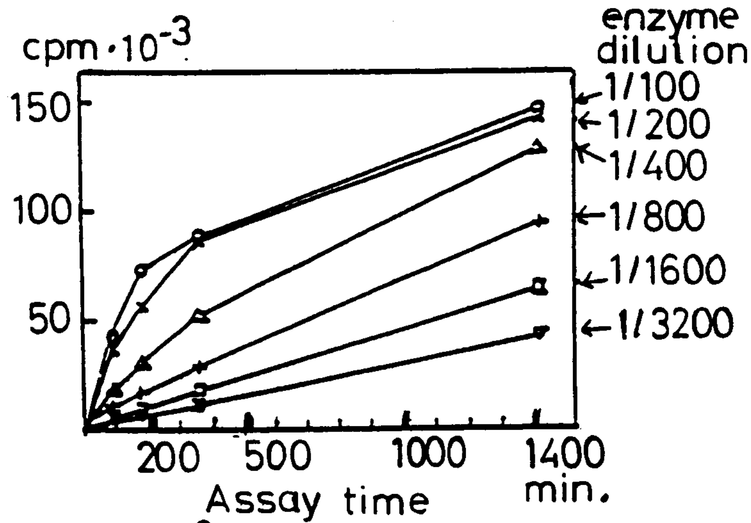


FIG.1B

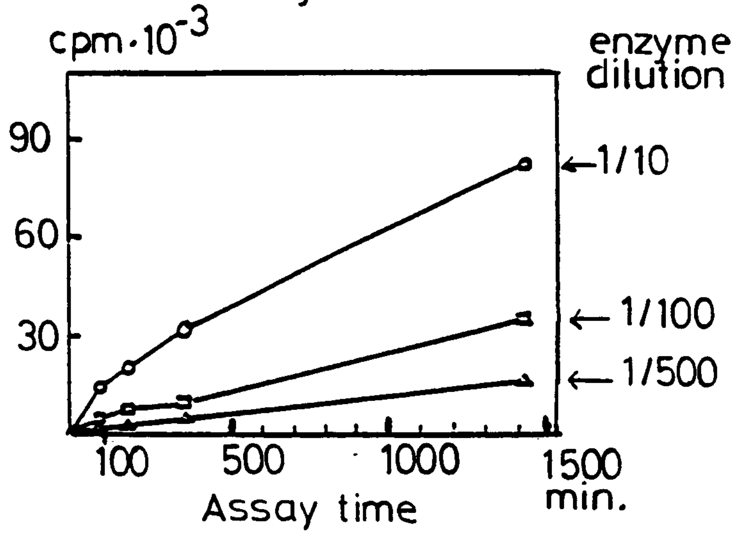


FIG.1C

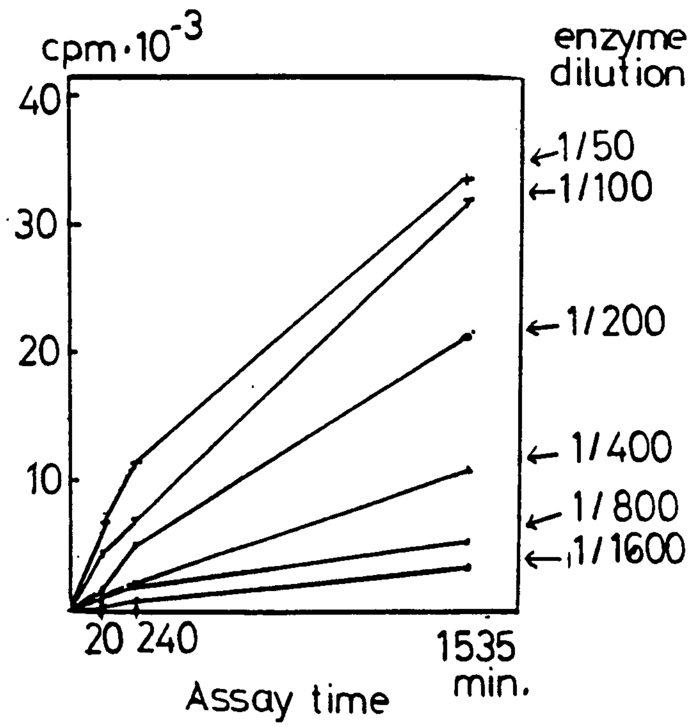

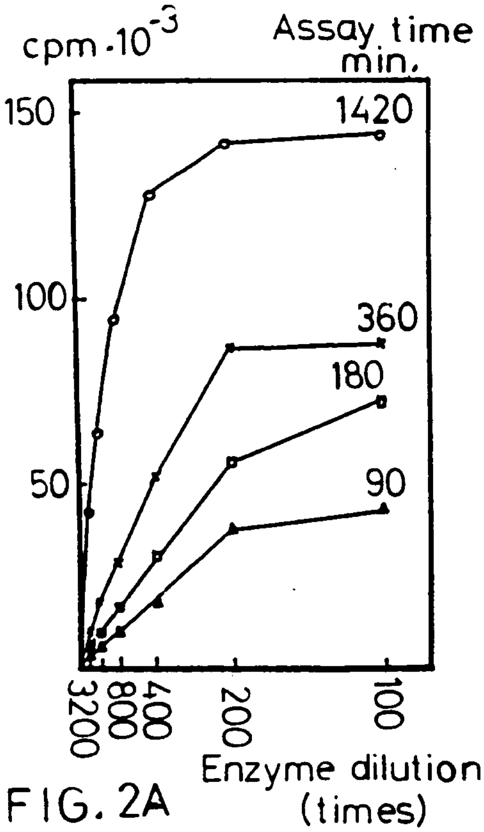


FIG. 2A

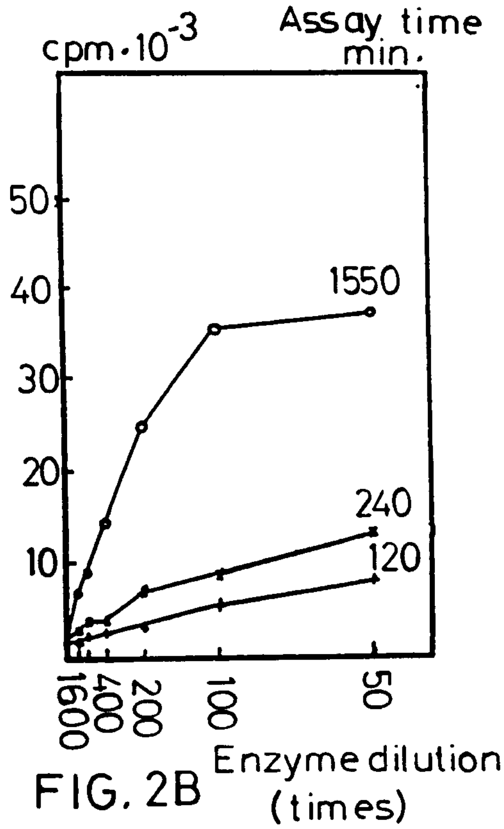


FIG. 2B

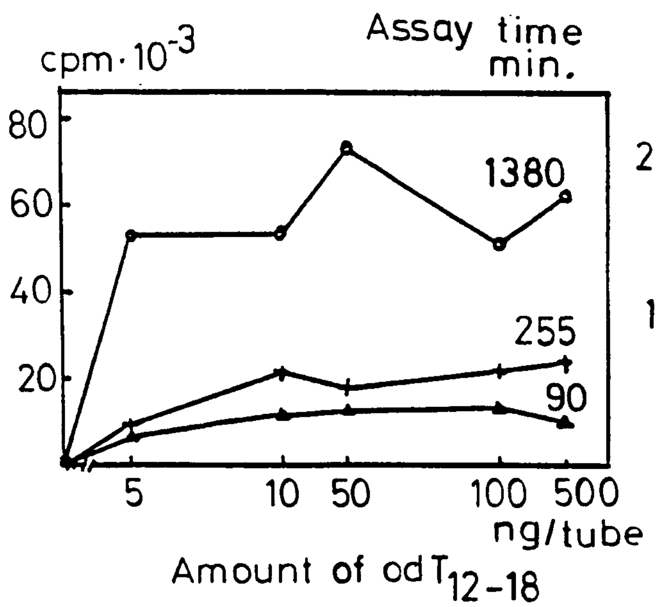


FIG. 3A

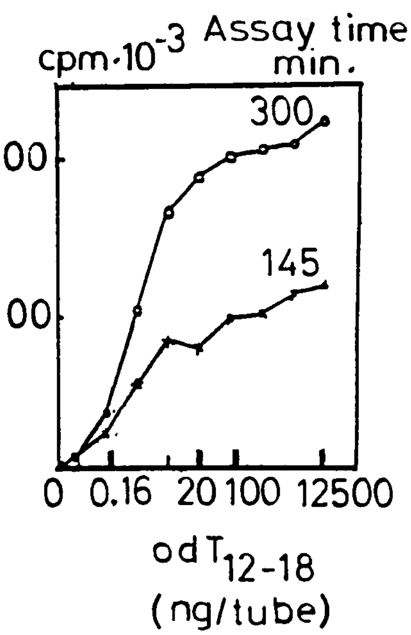


FIG. 3B

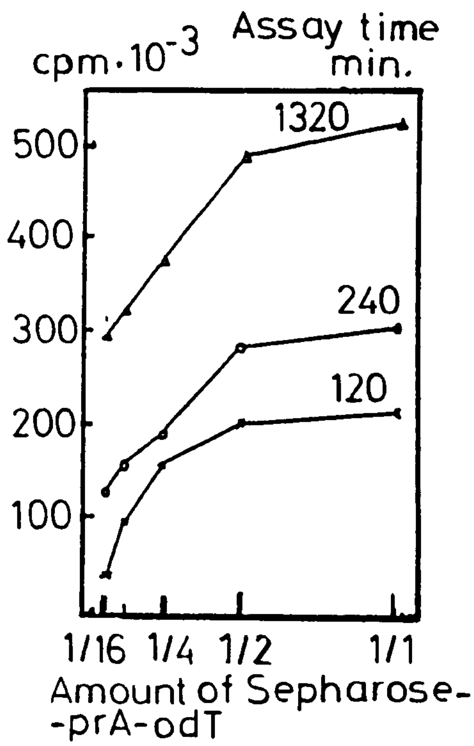


FIG. 4A

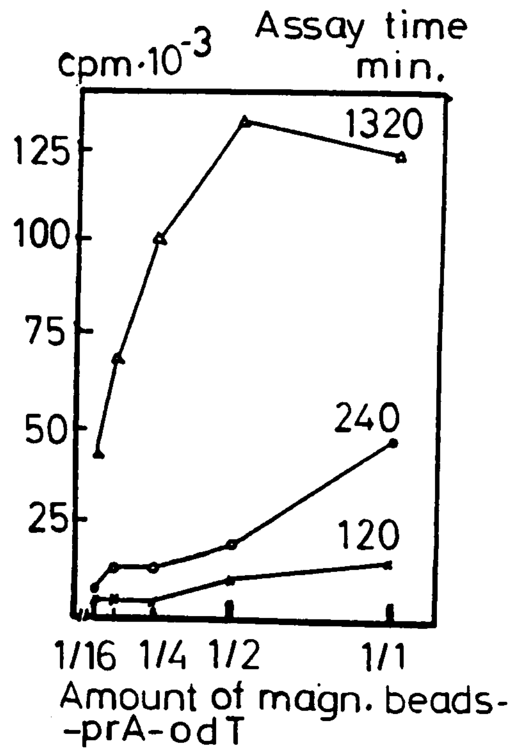


FIG. 4B

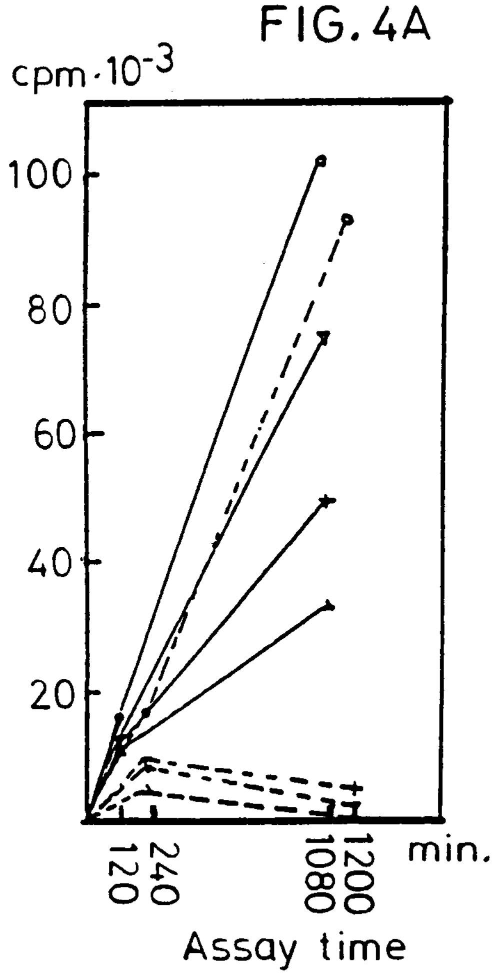


FIG. 5A

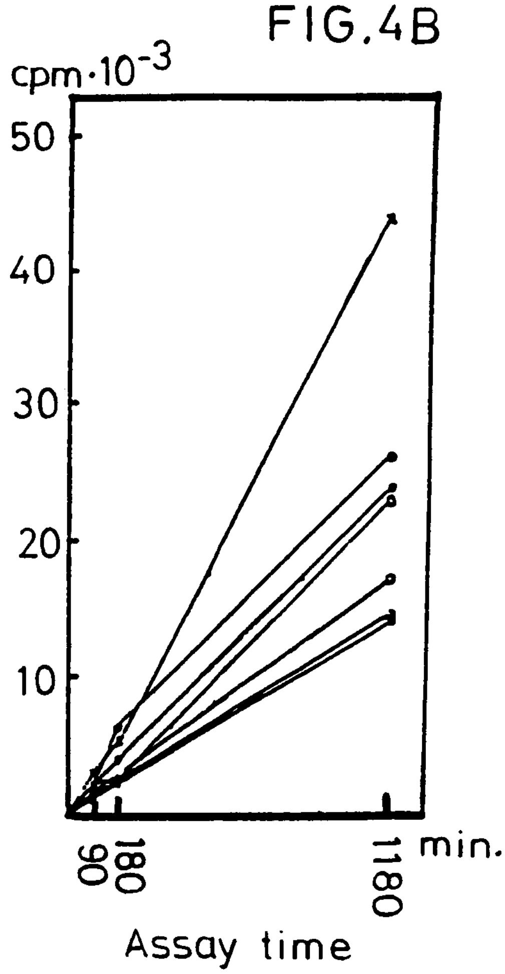


FIG. 5B

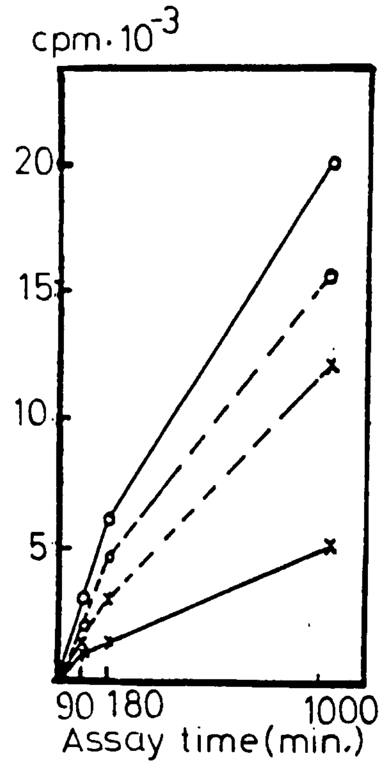


FIG. 6A

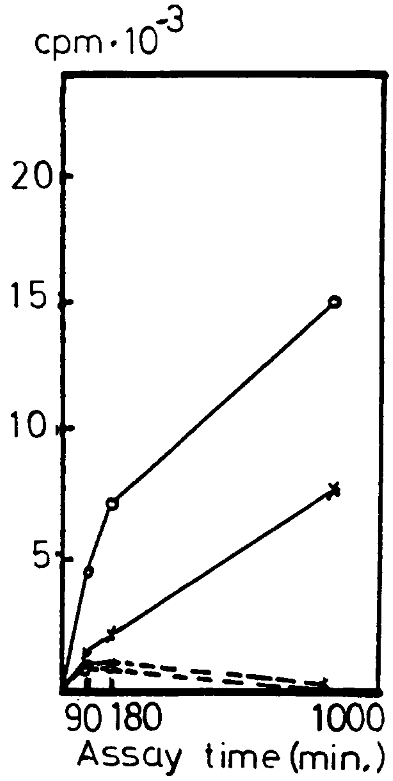


FIG. 6B

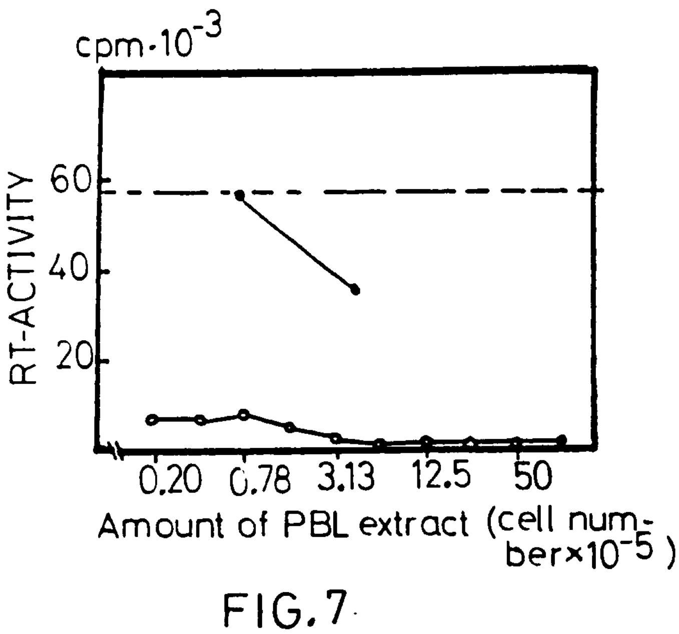


FIG. 7

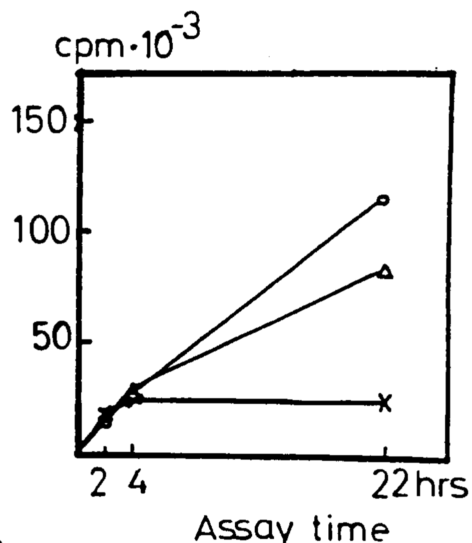


FIG. 8

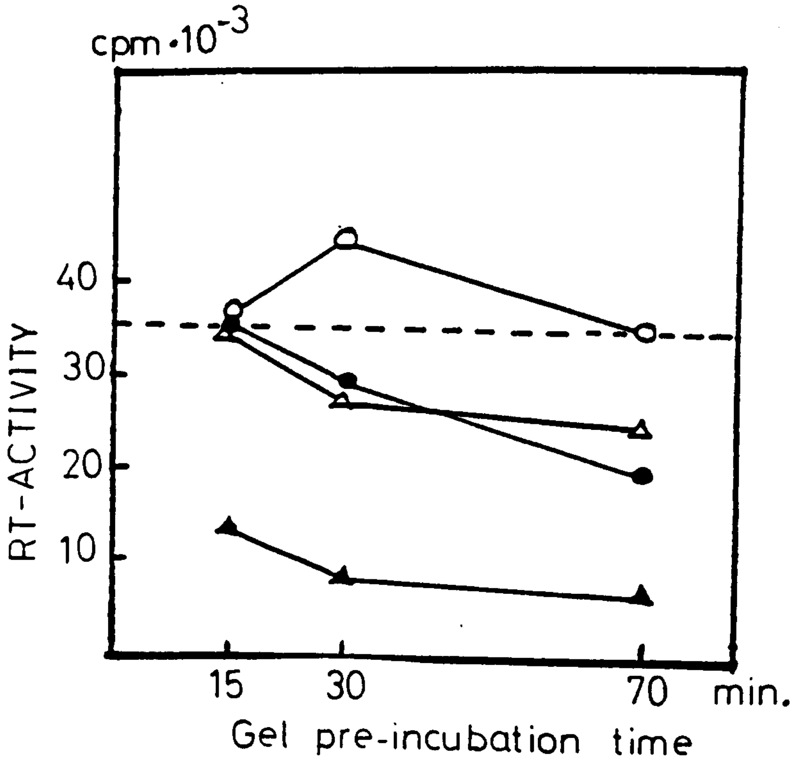


FIG.9

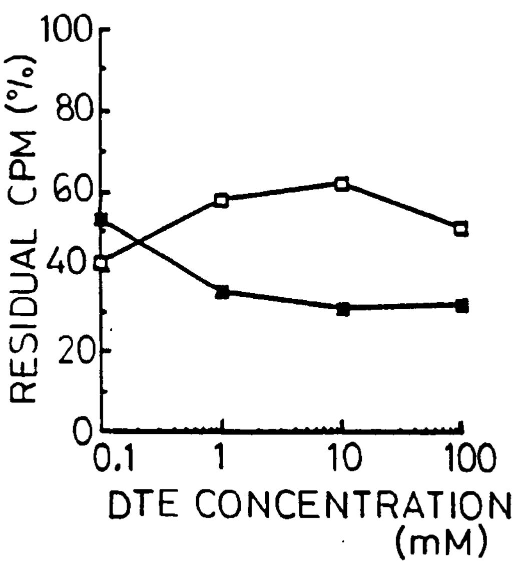


FIG.10

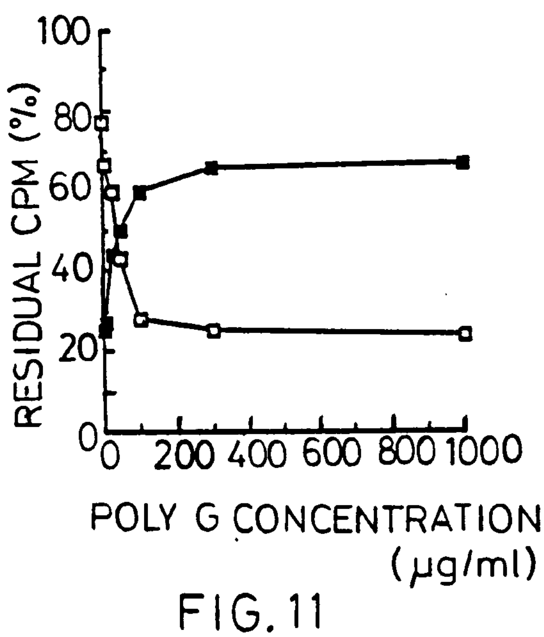


FIG.11

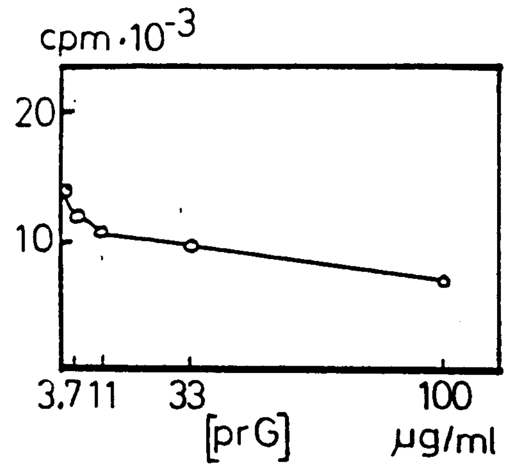


FIG. 12A

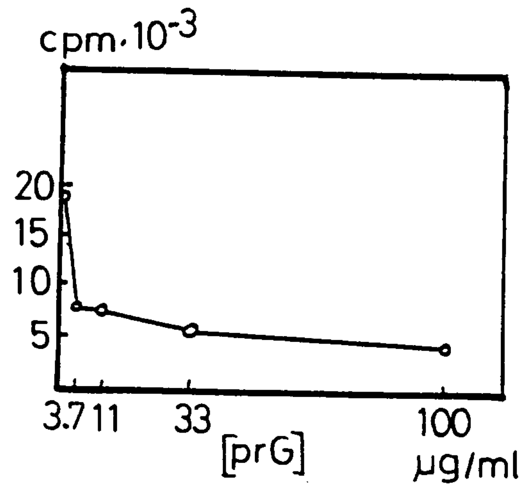


FIG. 12B

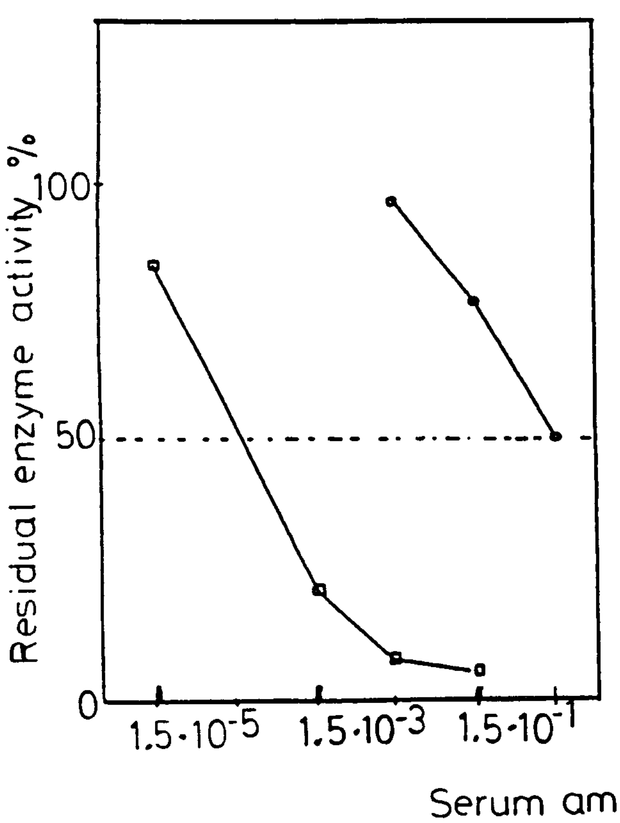


FIG. 13A

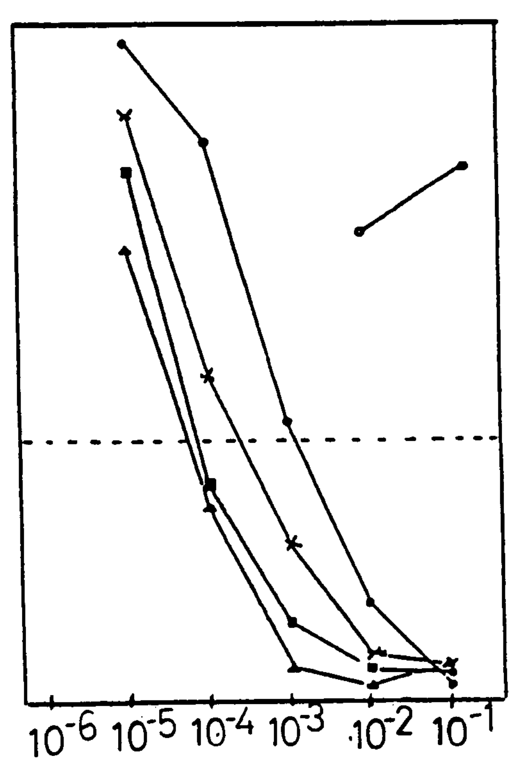


FIG. 13B

RT BLOCKING ANTIBODIES IN SERUMSAMPLES

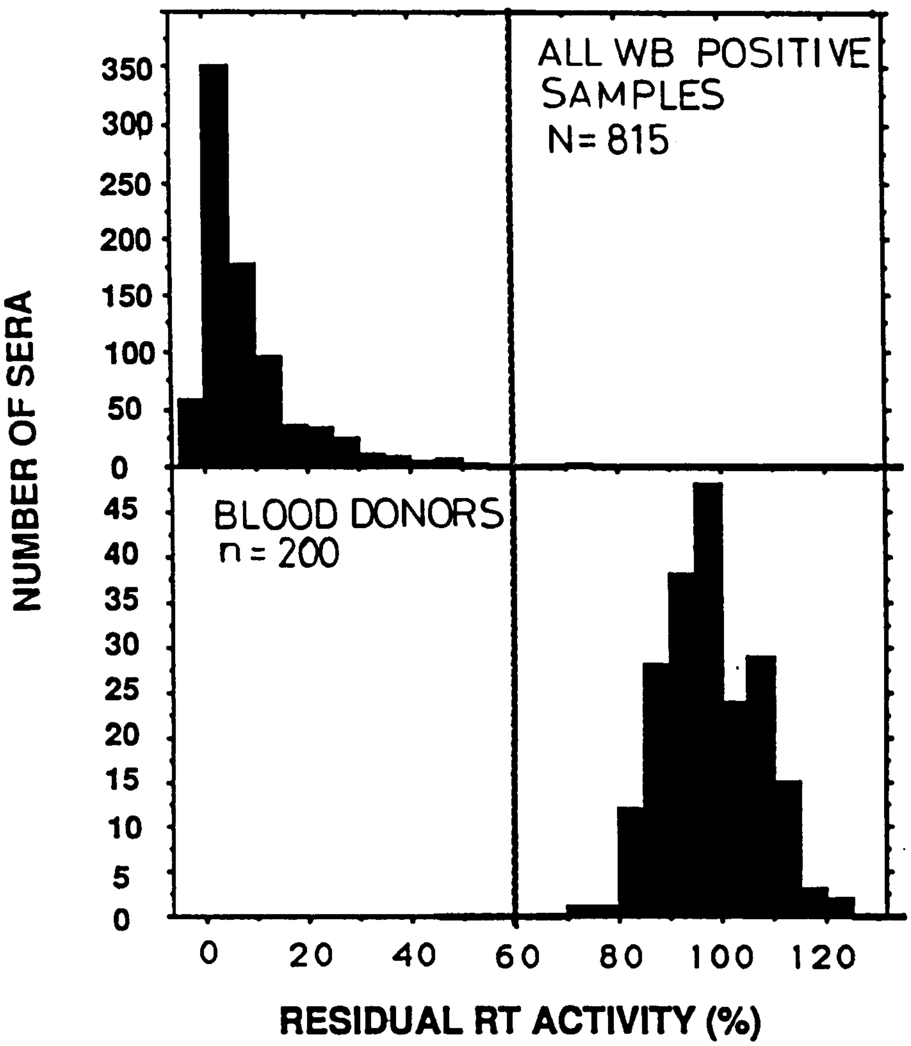


FIG.14A

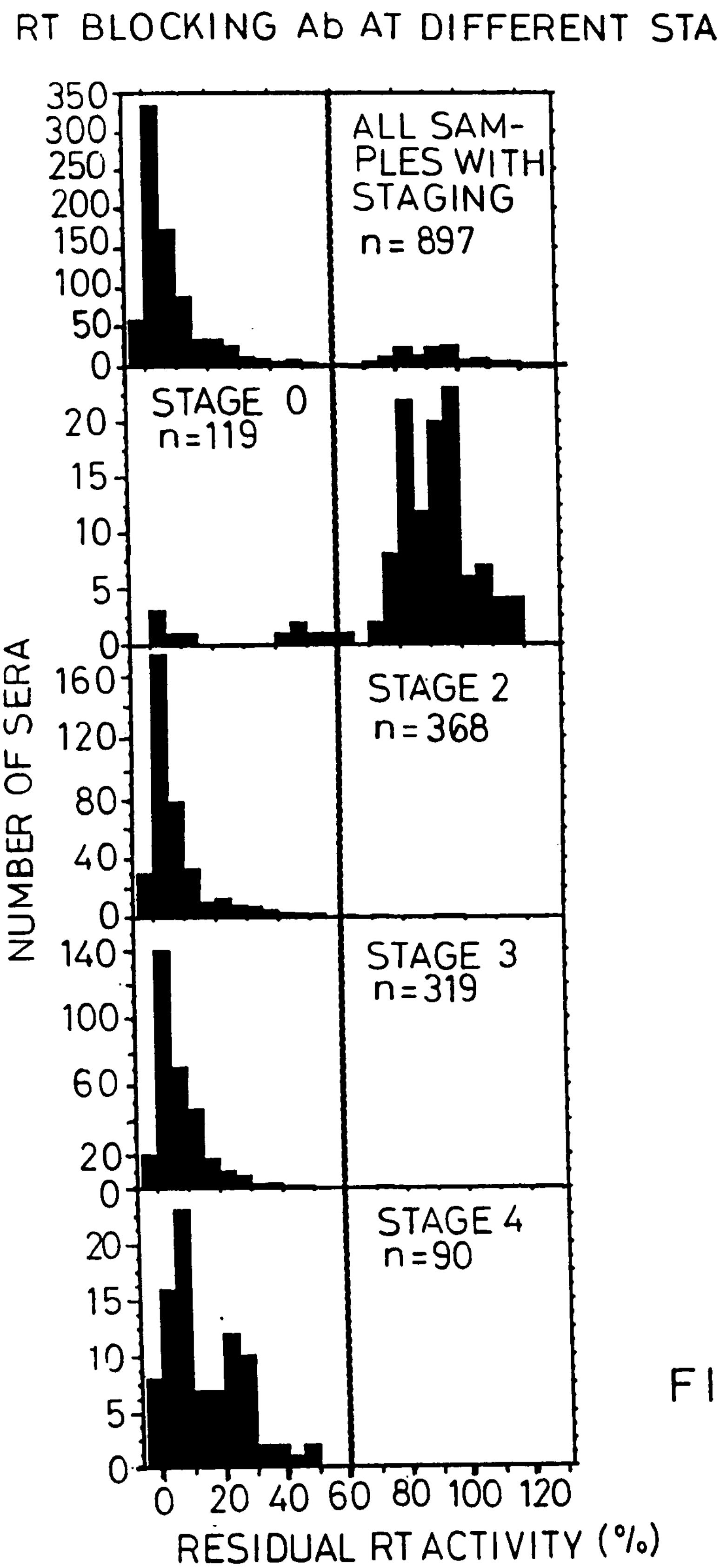
RT BLOCKING Ab AT DIFFERENT STAGES
NUMBER OF SERA
ALL SAMPLES WITH STAGING
n = 897
350
300
250
200
150
100
50
0
STAGE 0
n=119
20
15
10
5
0
STAGE 2
n = 368
160
120
80
40
0
STAGE 3
n=319
140
100
60
20
0
STAGE 4
n=90
20
15
10
5
0
0 20 40 60 80 100 120
RESIDUAL RT ACTIVITY (%)

FIG. 14B

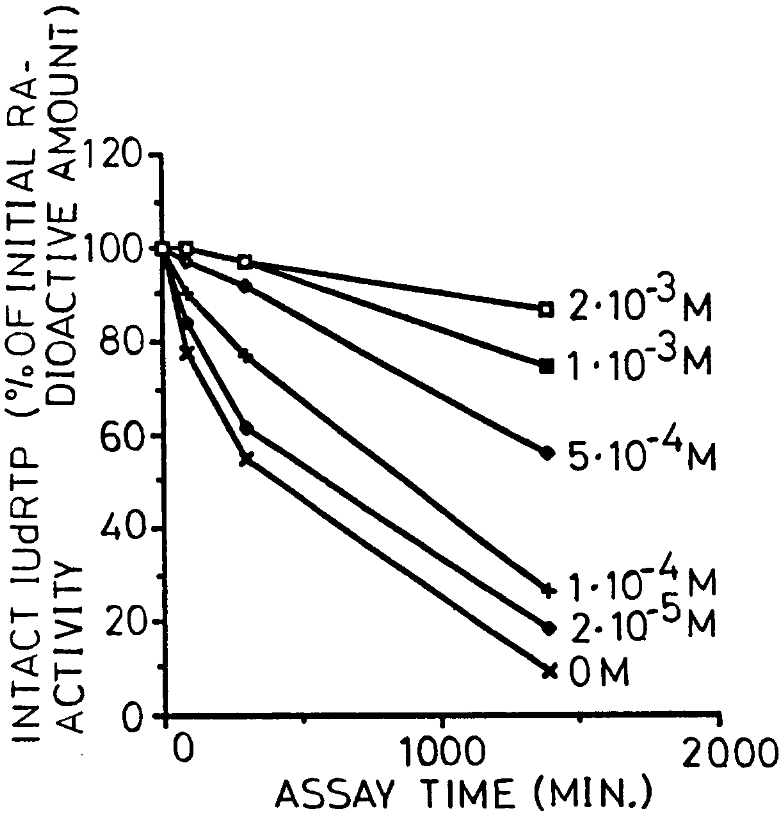


FIG. 15A

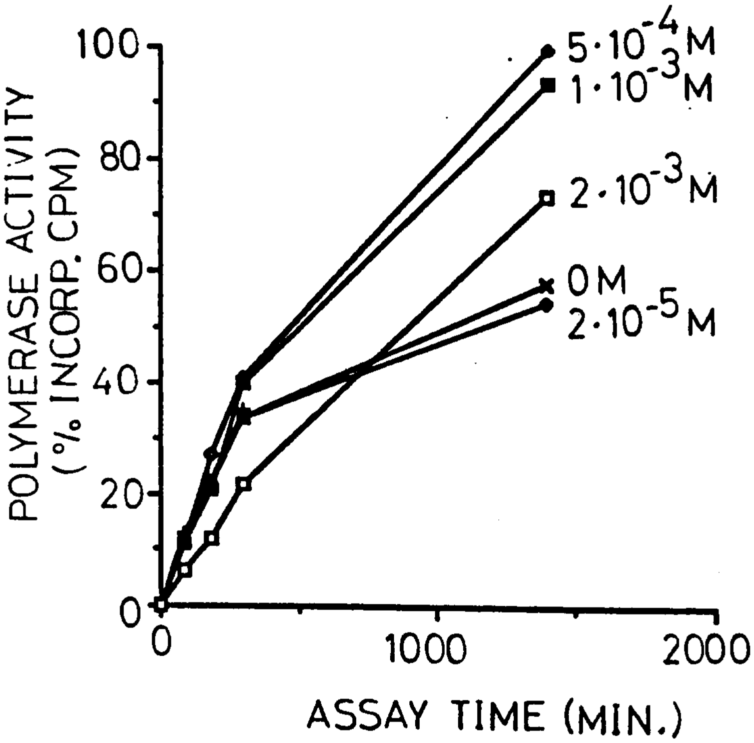


FIG. 15B